(19) Europäisches Patentamt
European Patent Office
Office européen des brevets

(11) Veröffentlichungsnummer: **0 247 320 B1**

(12) # EUROPÄISCHE PATENTSCHRIFT

(45) Veröffentlichungstag der Patentschrift: **27.12.90**

(51) Int. Cl.⁵: **C07D 307/58**, C07C 69/734, A61K 31/34

(21) Anmeldenummer: **87104404.6**

(22) Anmeldetag: **25.03.87**

(54) **5-Arylalkyl-4-alkoxy-2(5H)-furanone, Zwischenprodukte und Verfahren zu ihrer Herstellung sowie ihre Anwendung als therapeutische Wirkstoffe.**

(30) Priorität: **05.05.86 DE 3615157**

(43) Veröffentlichungstag der Anmeldung: **02.12.87 Patentblatt 87/49**

(45) Bekanntmachung des Hinweises auf die Patenterteilung: **27.12.90 Patentblatt 90/52**

(84) Benannte Vertragsstaaten: **AT BE CH DE FR GB IT LI NL SE**

(56) Entgegenhaltungen:
**TETRAHEDRON LETTERS, Nr. 18, April 1979, Seiten 1627-1630, London;A. PELTER: "The synthesis of piperolide and related Compounds".**
**TETRAHEDRON LETTERS, Band 23, Nr. 49, Dezember 1982, Seiten 5229-5232, London; A. PELTER et al.: "Carbon-Carbon Bond Formation by "Double Activation" The synthesis of piperolide and fadyenolide intermediates".**
**CHEMICAL ABSTRACTS, Band 90, Nr. 3, 15. Jan. 1979, Seite 619, Columbus, Ohio, USA; R.HAEUSEL et al."Behavior of 5-hydroxykawain derivatives against alkalis", Spalte 2, Z'fassung 227054.**
**JOURNAL OF THE CHEMICAL SOCIETY, Perkin Transaction I, Nr. 4, April 1981, Seiten 1173-1179, London; A.PELTER et al.: "The Carbon-13 Nuclear Magnetic Resonance Spectra of Tetronate and 2-Pyrone Derivatives".**
**TETRAHEDRON LETTERS, Band 23, Nr. 10, März 1982, Seiten 1793-1796, London; R.R. SCHMIDT "Beta- and Alpha-Lithiation of Methyl Beta-Methoxyacrylate: Efficient Synthesis of Alpha, Gamma-Substituted Methyl Tetronates- Structure of Aspertetronins and Gregatins" .47wassung Nr. 219687zeite 595, Spalte 1, Zusammenfassung Nr. 6362c 000**
**ANGEWANDTE CHEMIE, Band 93, Nr. 6/7, 1981, Seiten 587-593, Weinheim; H.J. BESTMANN " Ein neuer Weg zu 2-Ethoxybutadienen; Diels-Alder-Reaktion von 1,2-lambda5-Oxaphosphorinen".**
**BURGER'S MEDICINAL CHEMISTRY, 4.Ausgabe, Teil III, Kapitel 55, 1981, Wiley-Interscience, Seiten829-858; M.E. WOLFF: "Anticonvulsants".**

(73) Patentinhaber: **Dr. Willmar Schwabe GmbH & Co., Dr. Willmar-Schwabe-Strasse 4, D-7500 Karlsruhe 41(DE)**

(72) Erfinder: **Chatterjee,Dr. Shyam Sunder, Stettiner Str. 1, D-7500 Karlsruhe 1(DE)**
Erfinder: **Dr. Klessing, Klaus, Rheingoldstr. 3, D-7505 Ettlingen 5(DE)**

(74) Vertreter: **Bunke, Holger, Dr.rer.nat. Dipl.-Chem., Patentanwälte Prinz, Leiser, Bunke & Partner Manzingerweg 7, D-8000 München 60(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 247 320 B1

ACTORUM AG

## Beschreibung

Die Erfindung betrifft 5-Arylalkyl-4-alkoxy-2(5H)-furanone der allgemeinen Formel I

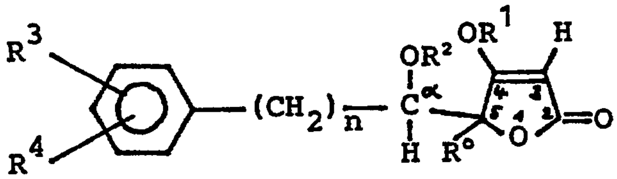

worin die Sauerstoffatome an C-5 und C-α relativ zueinander die threo-Position einnehmen und worin
n die Zahl 0, 1 oder 2,
$R^0$ ein Wasserstoffatom oder eine Niederalkylgruppe mit 1 bis 3 C-Atomen,
$R^1$ eine geradkettige oder verzweigte Alkylgruppe mit 1 bis 5 C-Atomen,
$R^2$ ein Wasserstoffatom, eine Niederalkylgruppe mit 1 bis 3 C-Atomen oder den Rest

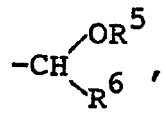

in welchem
$R^5$ eine Niederalkylgruppe mit 1 bis 5 C-Atomen, die Ethoxyethyl- oder Methoxyethylgruppe und
$R^6$ ein Wasserstoffatom, eine Niederalkylgruppe mit 1 bis 5 C-Atomen oder die Methoxymethylgruppe bedeuten,
$R^3$ und $R^4$ unabhängig voneinander ein Wasserstoff-, Fluor-, Chlor- oder Bromatom, eine Niederalkylgruppe mit 1 bis 3 C-Atomen, eine Perfluorniederalkylgruppe mit 1 bis 3 C-Atomen, die Difluormethoxy- oder Nitrogruppe
bedeuten, unter Ausschluß derjenigen Verbindungen der allgemeinen Formel I, worin
$R^2$ H oder $CH_3$ bedeutet, wenn
$n = 0$ oder 2, $R^0 = H$, $R^1 = CH_3$, $R^3 = H$ und $R^4$ - H ist.

Die Erfindung betrifft ferner Zwischenprodukte und Verfahren zur Herstellung der vorgenannten Verbindungen sowie die Anwendung dieser Verbindungen als therapeutische Wirkstoffe und damit auch Arzneimittel, die die genannten Verbindungen oder die durch Disclaimer vom Stoffschutz ausgenommenen Verbindungen enthalten.

Da die Verbindungen der allgemeinen Formel I mit den asymmetrischen Kohlenstoffatomen C-5 und C-α zwei Chiralitätszentren in ihrem Molekül enthalten, gibt es zwei Diastereomerenpaare, nämlich die beiden threo-Enantiomeren:

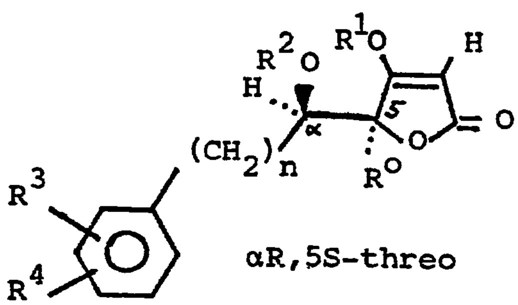

αR,5S-threo

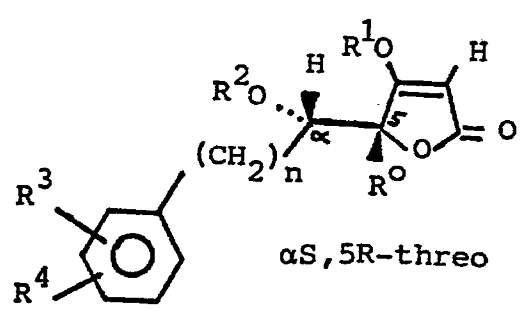

αS,5R-threo

und die beiden erythro-Enantiomeren:

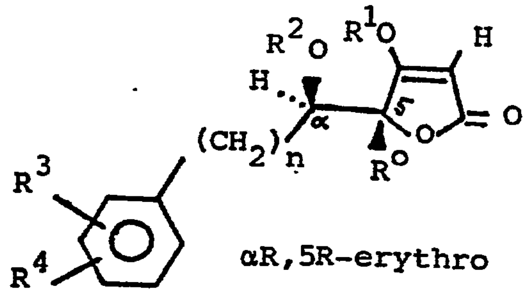

αR,5R-erythro

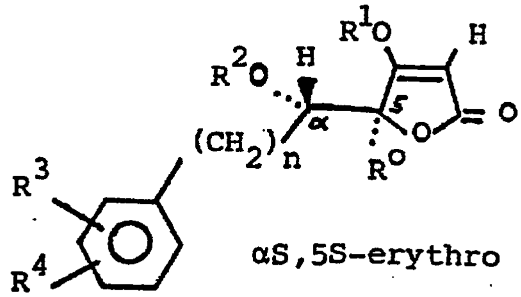

αS,5S-erythro

Die Erfindung befaßt sich ausschließlich mit den beiden threo-Enantiomeren, die in Form ihres Racemats oder ihrer beiden links- und rechtsdrehenden optischen Antipoden vorliegen können, sowie mit Verfahren, die stereoselektiv zu den threo-Enantiomeren führen.

Die durch Disclaimer vom Stoffschutz ausgenommenen Verbindungen sind bekannt:
4-Methoxy-5-phenylhydroxymethyl-2(5H)-furanon wurde von A. Pelter et al., Tetrahedron Lett, 1979, S. 1627-1630, und
4-Methoxy-5-[methoxy-(phenyl)-methyl)]-2(5H)furanon von A. Pelter et al., Tetrahedron Lett. 1982, S. 5229-5232, beschrieben;
4-Methoxy-5-(α-hydroxy-γ-phenylpropyl)-2(5H)-furanon und 4-Methoxy-5-(α-methoxy-γ-phenylpropyl)-2(5H)-furanon wurden von R. Hänsel et al., Z. Naturforsch. 33b, S. 1020-1025 (1978), beschrieben.

Über eine pharmakologische Wirksamkeit oder gar eine therapeutische Anwendbarkeit der bekannten Verbindungen berichten die genannten Autoren jedoch nichts.

Zur Herstellung der bekannten Verbindungen schlagen R. Hänsel et al. a.a.O. vor, aus der in Mexiko beheimateten Art piper sanctum 5,6-Z-Piperolid zu isolieren, das durch Photonenbestrahlung in das stereoisomere 5,6-E-Piperolid übergeführt werden kann. Durch katalytische Hydrierung läßt sich 5,6-E-Piperolid zu 5,6-threo-Tetrahydropiperolid (= 4-Methoxy-5-(α-methoxy-γ-phenylpropyl)-2(5H)-furanon) umsetzen. Die Gewinnung des Ausgangsmaterials, also des Naturstoffs 5,6-Z-Piperolid und dessen Umwandlung in 5,6-E-Piperolid ist jedoch sehr teuer und daher für eine kommerzielle Nutzung ungeeignet.

Ein anderes bekanntes Verfahren zur Herstellung der bekannten Verbindungen (vgl. A. Pelter et al., Tetrahedron Lett. 1979, S. 1627-1630) geht von Methyltetronat aus, dessen Herstellung aus der EP-PS 10 288 bekannt ist.

Hierbei wird Methyltetronat mit Lithiumdiisopropylamid unter Bildung eines Carbanions versetzt und dann bei -78°C mit Benzaldehyd oder Dihydrozimtaldehyd umgesetzt. Abgesehen davon, daß die Reaktionsbedingungen (-78°C!) für eine wirtschaftliche Herstellung in großem Maßstab ungeeignet sind, entstehen bei diesem Verfahren stets Gemische der threo- und erythro-Diastereomeren, so daß zur Abtrennung der threo-Verbindungen in reiner Form aufwendige Reinigungsoperationen erforderlich sind, sofern die Auftrennung überhaupt gelingt. Darüber hinaus ist die eingesetzte lithiumorganische Verbindung selbstentzündlich und das als Lösungsvermittler zugesetzte Hexamethylphorphorsäuretriamid (HMPA) kanzerogen.

Es sind noch eine Reihe von Varianten des von A. Pelter et al. beschriebenen Verfahrens bekannt, die jedoch alle die gleichen prinzipiellen Nachteile besitzen, da sie nicht stereoselektiv zu einem der beiden Diastereomerenpaare führen und wegen der erforderlichen tiefen Temperaturen sehr energieaufwendig sind.

Schließlich sind auch schon eine ganze Reihe von Verbindungen unterschiedlichster chemischer Konstitution mit anticonvulsiver und antiepileptischer Wirksamkeit bekannt (vgl. z.B. Ehrhart/Ruschig, Arzneimittel, Bd. 1, S. 177 ff., Verlag Chemie, Weinheim, 1972), zu denen insbesondere die Wirkstoffe Carbamazepin, Diazepam, Diphenylhydantoin, Ethosuximid, Phenobarbital und Valproinsäure gehören. Alle diese bekannten Anticonvulsiva/Antiepileptika weisen chronisch-toxische Nebenwirkungen in unterschiedlichem Maße auf, zu denen Exantheme, depressive Verstimmungen, Paranoia, Megaloblasten-Anämie, Schädigungen des blutbildenden Knochenmarks, Leberschädigungen u.a. gehören.

Es bestand deshalb ein Bedürfnis nach der Bereitstellung neuer pharmazeutischer Mittel mit anticonvulsiver und antiepileptischer Wirksamkeit, weil der Arzt nur so in die Lage versetzt wird, aus einem größeren Arzneimittelschatz Mittel auszuwählen, deren Wirkungs- und Nebenwirkungsspektren den physischen und psychischen Bedürfnissen des Patienten am ehesten gerecht werden.

Die der Erfindung zugrunde liegende Aufgabe besteht darin, dieses Bedürfnis nach der Bereitstellung neuer Verbindungen mit anticonvulsiver/antiepileptischer Wirksamkeit zu befriedigen. Der Erfindung liegt ferner die Aufgabe zugrunde, Verfahren zur Herstellung solcher Verbindungen mit antikonvulsiver/antiepileptischer Wirksamkeit zu schaffen, mit denen es gelingt, stereoselektiv die threo-Enantiomeren entweder in Form ihrer Racemate oder in Form ihrer einzelnen optischen Antipoden zu synthetisieren.

Die Lösung dieser Aufgabe besteht in der Schaffung und Bereitstellung der erfindungsgemäßen Stoffe mit antikonvulsiver/antiepileptischer Wirksamkeit sowie der diese Verbindungen enthaltenden Arzneimittel und in der Schaffung der erfindungsgemäßen Verfahren zur Herstellung der neuen Stoffe.

Gegenstand der Erfindung sind somit zunächst die eingangs genannten 5-Arylalkyl-4-alkoxy-2(5H)-furanone der allgemeinen Formel I im Umfang des Patentanspruchs 1, und zwar sowohl in Form ihrer DL-Racemate als auch in Form der beiden optischen Antipoden (D- und L-Form), die sich aus den Racematen nach dem Fachmann geläufigen Methoden der Racematspaltung in optisch reiner Form herstellen lassen.

Die erfindungsgemäßen Verbindungen der threo-Reihe weisen eine überraschend gute antikonvulsive/antiepileptische Wirksamkeit auf, während die entsprechenden Verbindungen de erythro-Reihe in vergleichbarer Dosierung unwirksam sind. Die erfindungsgemäßen Verbindungen sind zur Behandlung der Epilepsie des Menschen geeignet.

Gegenstand der Erfindung sind insbesondere:

- 4-Methoxy-5-(phenylhydroxymethyl)-2(5H)-furanone der allgemeinen Formel II

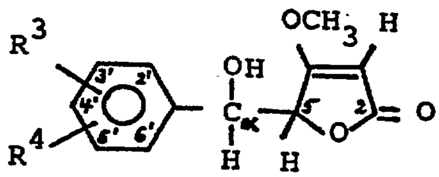

(II)

worin die Sauerstoffatome an C-5 und C-α relativ zueinander die threo-Position einnehmen und worin einer der beiden Reste $R^3$ und $R^4$ ein Wasserstoffatom und der andere ein in 2'-Stellung befindliches Fluor-, Chlor- oder Bromatom oder eine in 2'-Stellung befindliche Methyl-, Trifluormethyl- oder Nitrogruppe bedeuten;

- 4-Methoxy-5-(phenylhydroxymethyl)-2(5H)-furanone der allgemeinen Formel II, worin die Sauerstoffatome an C-5 und C-α relativ zueinander die threo-Position einnehmen und worin einer der beiden Reste $R^3$ und $R^4$

-- ein Fluor-, Chlor- oder Bromatom oder eine Trifluormethylgruppe, jeweils in 2'-Stellung,

und der andere

-- ein Chlor- oder Bromatom oder eine Trifluormethylgruppe, jeweils in 4'-, 5'- oder 6'-Stellung,

bedeuten;

- 4-Methoxy-5-[methoxy-(phenyl)-methyl]-2(5H)-furanone der allgemeinen Formel III

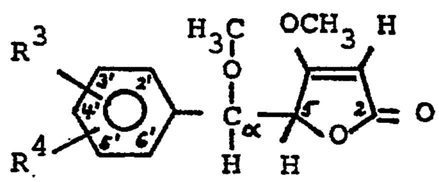

(III)

worin die Sauerstoffatome an C-5 und C-α relativ zueinander die threo-Position einnehmen und worin $R^3$ und $R^4$ die in Anspruch 1 angegebene Bedeutung besitzen, jedoch unter Ausschluß derjenigen Verbindungen der allgemeinen Formel III, worin beide Reste $R^3$ und $R^4$ jeweils ein Wasserstoffatom bedeuten;

- 4-Methoxy-5-(phenylmethyl)-2(5H)-furanone der allgemeinen Formel IV

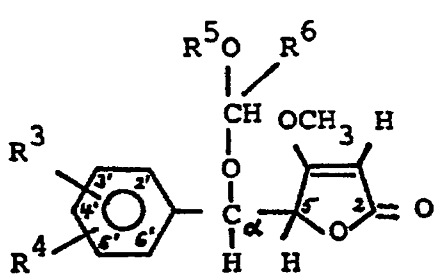

(IV)

worin die Sauerstoffatome an C-5 und C-α relativ zueinander die threo-Position einnehmen und worin

$R^5$ eine Methyl- oder Methoxyethylgruppe und
$R^3$, $R^4$ und $R^6$ die in Anspruch 1 angegebene Bedeutung besitzen.

Wegen ihrer guten antikonvulsiven/antiepileptischen Wirksamkeit werden die folgenden erfindungsgemäßen Verbindungen besonders bevorzugt:
threo-5-(2'-Chlorphenylhydroxymethyl)-4-methoxy-2(5H)-furanon;
threo-5-(2'-Bromphenylhydroxymethyl)-4-methoxy-2(5H)-furanon;
threo-5-(2'-Fluorphenylhydroxymethyl)-4-methoxy-2(5H)-furanon;
threo-5-(2'-Trifluormethyl-phenyl-hydroxymethyl)-4-methoxy-2(5H)-furanon;
threo-5-(2',5'-Dichlorphenyl)-4-methoxy-2(5H)-furanon;
threo-5(2',4'-Dichlorphenyl)-4-methoxy-2(5H)-furanon;
threo-4-Methoxy-5-[methoxymethoxy-(2'-chlorphenyl)-methyl]-2(5H)-furanon;
threo-4-Methoxy-5-[methoxymethoxy-(2'-bromphenyl)-methyl]-2(5H)-furanon;
threo-4-Methoxy-5-[methoxymethoxy-(2'-fluorphenyl)-methyl]-2(5H)-furanon;
threo-4-Methoxy-5-[methoxymethoxy-(2'-trifluormethylphenyl)-methyl]-2(5H)-furanon;
threo-4-Methoxy-5-[methoxy-(2'-chlorphenyl)-methyl-]-2(5H)-furanon.

Gegenstand der Erfindung sind ferner 3-Alkoxy-5-(subst.)-phenyl-2(E),4(E)-pentadienoate der allgemeinen Formel IX

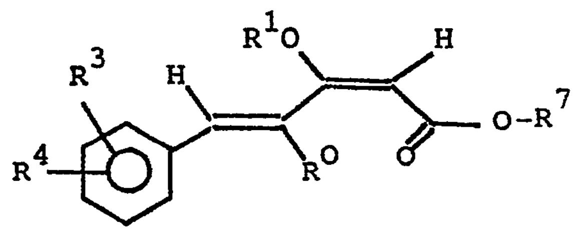
(IX)

worin
$R^0$, $R^1$, $R^3$ und $R^4$ die in Anspruch 1 angegebenen Bedeutungen besitzen und
$R^7$ ein Wasserstoffatom, eine geradkettige oder verzweigte Alkylgruppe mit 1 bis 10 C-Atomen oder eine Aralkylgruppe mit 7 bis 10 C-Atomen ist,
mit Ausnahme derjenigen Verbindung, worin $R^0$ = H, $R^1$ = Ethyl,
$R^3$ = H, $R^4$ = H und $R^7$ = Ethyl.

Die vom Schutz ausgenommene Verbindung der allgemeinen Formel IX ist bekannt aus Angew. Chemie 93 (1981), Nr. 6/7, S. 588, Tab. 1, Verb. (11c). Die bekannte Verbindung wird zusammen mit weiteren 1-Acyl-2-ethoxybutadienen durch eine Wittig-Reaktion aus Acylphosphoranen hergestellt und kann zu γ, δ-ungesättigten 1,3-Dicarbonylverbindungen und Derivaten des 2,3-Dihydro-4-pyrons weiter umgesetzt werden.

Die neuen Verbindungen der allgemeinen Formel IX sind reaktive Zwischenprodukte, die zur Herstellung der erfindungsgemäßen Verbindungen der allgemeinen Formel I verwendet werden können.

Bei einer bevorzugten Gruppe der vorgenannten reaktiven Zwischenprodukte der allgemeinen Formel IX ist $R^7$ ein Wasserstoffatom, eine geradkettige oder verzweigte Alkylgruppe mit 1 bis 4 C-Atomen oder eine Benzylgruppe.

Gegenstand der Erfindung ist schließlich ein Verfahren zur Herstellung derjenigen Verbindungen der allgemeinen Formel I, worin n = 0 ist, das durch die Kombination folgender Verfahrensstufen gekennzeichnet ist:

(A) Ein Benzaldehyd der allgemeinen Formel VI

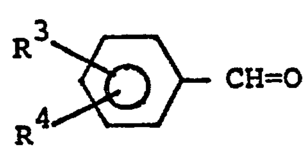
(VI)

worin $R^3$ und $R^4$ die im Zusammenhang mit der Erläuterung der Formel I genannten Bedeutungen besitzen, wird mit einem 3-Alkoxy-2(E)-alkensäure-niederalkylester der allgemeinen Formel VII

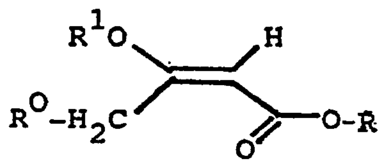

(VII)

worin $R^0$ und $R^1$ die im Zusammenhang mit der Erläuterung der allgemeinen Formel I genannten Bedeutungen besitzen und R eine Niedrigalkylgruppe mit 1 bis 4 C-Atomen ist, unter hydrolytischer Abspaltung des Restes R stereoselektiv zur entsprechend substituierten 2(E),4(E)-Pentadiensäure der allgemeinen Formel VIII

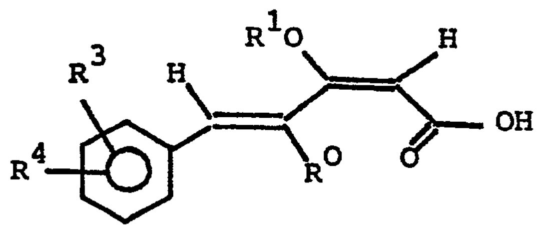

(VIII)

worin $R^0$, $R^1$, $R^3$ und $R^4$ die genannten Bedeutungen besitzen, kondensiert;

(B) die so entstandene Pentadiensäure wird in einen Ester der allgemeinen Formel IX

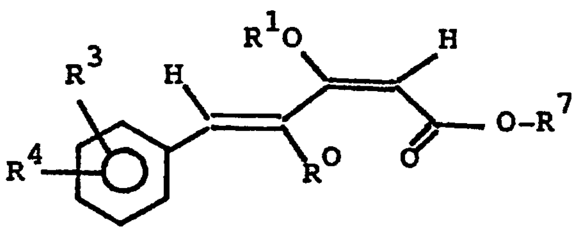

(IX)

worin $R^7$ eine geradkettige oder verzweigte Alkylgruppe mit 1 bis 10 C-Atomen oder eine Aralkylgruppe mit 7 bis 10 C-Atomen ist, übergeführt;

(C) der Ester der allgemeinen Formel IX wird durch cis-Dihydroxylierung mittels katalytischer Mengen $OsO_4$ und eines Oxidationsmittels zum entsprechenden 3-Alkoxy-4,5(threo)-dihydroxy-5-($R^3$,$R^4$-subst.)phenyl-2(E)-pentensäureester der allgemeinen Formel XI umgesetzt

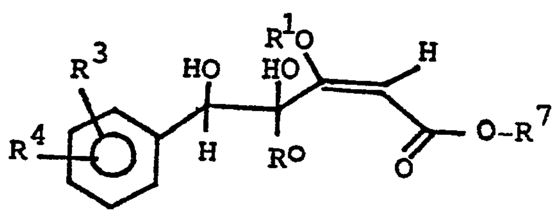

(XI)

(D) woraus unter 1.4-Eliminierung des Alkohols $R^7OH$ das entsprechende threo-4-Alkoxy-5-[($R^3$,$R^4$-subst.)phenylhydroxymethyl]-2(5H)-furanon der allgemeinen Formel I mit $R^2$ = H gebildet wird,

(E) welches anschließend gegebenenfalls mit einer reaktiven Verbindung der allgemeinen Formel $R^2$-X, worin $R^2$ die im Zusammenhang mit der Erläuterung der allgemeinen Formel I genannte Bedeutung, mit Ausnahme von Wasserstoff, besitzt und X eine reaktive Gruppe ist, z.B. Halogen oder Alkoxy, unter Abspaltung von X-H zur entsprechenden Verbindung der allgemeinen Formel I mit $R^2 \neq$ H umgesetzt wird.

Vorteilhafte Ausführungsformen des erfindungsgemäßen Verfahrens bestehen darin,
- daß die Kondensation der Stufe (A) in einem dipolaren aprotischen Lösungsmittel und in basischem Milieu durchgeführt wird;
- daß die Kondensation in Dimethylsulfoxid/Wasser unter Zugabe von Alkalimetallhydroxid, quartären Ammoniumbasen oder deren Gemischen durchgeführt wird;
- daß die Kondensation unter Inertgasatmosphäre bei einer Temperatur zwischen etwa 70 und 140°C durchgeführt wird.

- daß in Stufe (C) Alkylhydroperoxide, tertiäre Amin-N-oxide oder Chlorate als Oxidationsmittel verwendet werden;
- daß die Dihydroxylierung in Stufe (C) in Gegenwart einer quartären Ammoniumbase oder eines quartären Ammoniumsalzes durchgeführt wird.

Eine weitere Ausführungsform des erfindungsgemäßen Verfahrens ist dadurch gekennzeichnet, daß die in Stufe (A) gebildete Pentadiensäure der allgemeinen Formel VIII der cis-Dihydroxylierung gemäß Stufe (C) unter Bildung der entsprechend substituierten Pentensäure der allgemeinen Formel XI mit $R^7$ = H unterworfen wird, wonach die Pentensäure gemäß Stufe (D) unter 1.5-Eliminierung von $H_2O$ zum entsprechend substituierten Furanon der allgemeinen Formel I mit $R^2$ = H umgesetzt wird, welches anschließend gegebenenfalls gemäß Stufe (E) zu einer Verbindung der allgemeinen Formel I mit $R^2 \neq H$ umgesetzt wird.

Das erfindungsgemäße Verfahren zur Herstellung derjenigen Verbindungen der allgemeinen Formel I, worin n = 0 ist, wird nachstehend noch näher anhand der Zeichnung (Formelschema) erläutert:

Verfahrensstufe A

Zur Synthese der 3-Alkoxy-5-($R^3$,$R^4$-subst.)phenyl-2(E), 4(E)-pentadiensäuren der allgemeinen Formel VIII kondensiert man die Benzaldehyde der allgemeinen Formel VI mit den 3-Alkoxy-2(E)-alkensäureniederalkylestern der allgemeinen Formel VII, vorzugsweise in einem dipolaren aprotischen Lösungsmittel, insbesondere Dimethylsulfoxid, bei erhöhter Temperatur und unter Zugabe von Alkalimetallhydroxiden und quartären Ammoniumbasen oder deren wäßrigen Lösungen und fällt nach beendeter Kondensation und Abkühlung des Reaktionsgemischs die 3-Alkoxy-5-($R^3$,$R^4$-subst.)phenyl-2(E),4(E)-pentadiensäuren durch Verdünnen mit Wasser und Ansäuern aus.

Besonders gute Ausbeuten werden bei Einhaltung folgender Reaktionsbedingungen erzielt: Zu 1 Mol Aldehyd der Formel VI fügt man 100 bis 500 ml Dimethylsulfoxid und 0,5 bis 2 Mol 3-Alkoxy-2(E)-alkensäure-niederalkylester der Formel VII sowie 0,01 bis 1 Mol einer quartären Ammoniumbase oder eines quartären Ammoniumsalzes, gegebenenfalls in Form einer wäßrigen Lösung, hinzu.

Unter einer Inertgasatmosphäre, beispielsweise unter Stickstoff oder Argon, erhitzt man das Reaktionsgemisch auf eine Temperatur zwischen etwa 70 und 140°C, vorzugsweise etwa 90 bis 110°C, und fügt mindestens eine der Menge des eingesetzten Esters der Formel VII äquimolare Menge Alkalimetallhydroxid, gegebenenfalls in Form einer konzentrierten wäßrigen Lösung, hinzu und rührt das Gemisch vorzugsweise etwa 2 bis 12 Stunden lang bei der genannten Temperatur.

Nach Abkühlen auf Raumtemperatur werden die Pentadiensäuren der Formel VIII durch Verdünnen mit Wasser und Ansäuern auf einen pH-Wert von etwa 1 bis 4 ausgefällt, abfiltriert, mit Wasser und anschießend mit einem niederen Alkohol gewaschen und gegebenenfalls getrocknet.

Als Benzaldehyde der allgemeinen Formel VI, worin $R^3$ und $R^4$ unabhängig voneinander Wasserstoff, Fluor, Chlor, Brom, $C_1$- bis $C_3$-Niederalkyl, $C_1$- bis $C_3$-Perfluorniederalkyl, Difluormethoxy oder Nitro bedeuten, können beispielsweise die folgenden Verbindungen eingesetzt werden:

Benzaldehyd,in 2-, 3- oder 4-Stellung durch Fluor, Chlor, Brom, Methyl, Trifluormethyl, Difluormethoxy oder Nitro substituierte Benzaldehyde, 2,4-, 2,5-, 2,6-, 3,4- oder 3,5-Dichlorbenzaldehyd, 2,4- oder 2,5-Dibrombenzaldehyd, 2,4-, 2,5- oder 3,5-bis-(Trifluormethyl)-benzaldehyd, 2-Chlor-4-brombenzaldehyd, 2-Chlor-5-brombenzaldehyd, 2-Chlor-4-trifluormethylbenzaldehyd, 2-Chlor-5-trifluormethylbenzaldehyd, 2-Brom-5-trifluormethylbenzaldehyd, 2-Trifluormethyl-4-chlorbenzaldehyd, 2-Trifluormethyl-5-chlorbenzaldehyd, 2-Fluor-4-chlorbenzaldehyd, 2-Fluor-5-trifluormethylbenzaldehyd etc.

Bevorzugt werden Benzaldehyd und solche monosubstituierten Benzaldehyde verwendet, die in 2-Stellung einen der Rest $R^3$ bzw. $R^4$ tragen. Bevorzugt werden außerdem solche disubstituierten Benzaldehyde verwendet, die in 2-Stellung den Rest Fluor, Chlor, Brom oder Perfluoralkyl und in 4- oder 5-Stellung den Rest Chlor, Brom oder Trifluormethyl tragen.

Die Benzaldehyde der allgemeinen Formel VI sind bekannt oder können nach bekannten Methoden hergestellt werden (vgl. Methodicum chimicum, Bd. 5, S. 203-336, Georg Thieme Verlag, Stuttgart, 1975).

Die 3-Alkoxy-2(E)-alkensäure-niederalkylester der allgemeinen Formel VII, worin "Niederalkyl" einen Alkylrest mit 1 bis 5 C-Atomen und $R^1$ eine geradkettige oder verzweigte Alkylgruppe mit 1 bis 5 C-Atomen bedeutet, sind ebenfalls bekannt oder können nach bekannten Methoden hergestellt werden. Bevorzugte Ester der allgemeinen Formel VII sind diejenigen Methyl- oder Ethylester, in denen $R^1$ eine Alkylgruppe mit 1 bis 3 C-Atomen bedeutet, also beispielsweise 3-Methoxy-2(E)-butensäuremethyl- oder -ethylester, 3-Ethoxy-2(E)-butensäuremethyl- oder -ethylester, 3-Propoxy-2(E)-butensäuremethyl- oder -ethylester, 3-Isopropoxy-2(E)-butensäuremethyl- oder -ethylester sowie die homologen 3-Alkoxy-2(E)-penten-, -hexen- oder -heptensäureester. Besonders bevorzugt sind 3-Methoxy-2(E)-dbutensäureethylester und 3-Ethoxy-2(E)-butensäureethylester.

Zur Erzielung hoher Ausbeuten werden bei der Kondensation in Stufe A des erfindungsgemäßen Verfahrens quartäre Ammoniumbasen, gegebenenfalls in Form wäßriger Lösungen, als Katalysatoren zugesetzt. Hierzu eignen sich besonders die als Phasentransferkatalysatoren bekannten quartären Tetraalkyl- oder Trialkylphenyl- oder Trialkylbenzylammoniumhydroxide, beispielsweise Tetraethyl- oder Tetrabutylammoniumhydroxid, Benzyltriethyl- oder -trimethylammoniumhydroxid oder Dodecyltrimethylammoniumhydroxid. Anstelle der quartären Ammoniumbasen können auch deren Salze mit Mineralsäuren, beispielsweise deren Chloride, Sulfate, Hydrogensulfate oder Bromide, unter Zusatz äquivalenter Mengen konzentrierter Kali- oder Natronlauge, eingesetzt werden.

Die Kondensation in Stufe A des erfindungsgemäßen Verfahrens führt unter gleichzeitiger Esterhydrolyse stets zu den 4,5-trans-konfigurierten Pentadiensäurederivaten der allgemeinen Formel VIII, was mit Hilfe der üblichen physikalischen Methoden nachgewiesen werden konnte. Wenn $R^0$ - H ist, betragen beispielsweise die Kopplungskonstanten der H-4- und H-5-Protonen im $^1$H-NMR-Spektrum etwa 15 bis 17 Hz.

Verfahrensstufe B

Die Pentadiensäurederivate der allgemeinen Formel VIII werden in an sich bekannter Weise in die Ester der allgemeinen Formel IX, worin $R^7$ einen $C_1$- bis $C_{10}$-Alkylrest oder einen $C_7$- bis $C_{10}$-Arylalkylrest bedeutet, übergeführt (vgl. beispielsweise Methodicum Chemicum, a.a.O., Bd. 5, S. 637-677 (1975)).

Verfahrenstufe C-1

Zur selektiven cis-Dihydroxylierung der C-4/C-5-Doppelbindung werden die Pentadiensäureester der allgemeinen Formel IX in Gegenwart katalytischer Mengen Osmiumtetroxid in einem geeigneten inerten Lösungsmittel mit einem Oxidationsmittel umgesetzt. Eine Übersicht über derartige, an sich bekannte Oxidationsverfahren findet sich bei M. Schröder, Chem. Rev. 80, S. 187-213(1980).

Als Oxidationsmittel wird vorzugsweise ein Alkylhydroperoxid oder ein aliphatisches, cycloaliphatisches oder heterocycloaliphatisches Amin-N-oxid, beispielsweise N-Methylmorpholin-N-oxid, vorzugsweise in Form einer konzentrierten wäßrigen Lösung, verwendet. Als inerte Lösungsmittel für die Oxidationsreaktion können tertiäre Alkanole, niedere Alkanone, niedere Alkylalkanoate oder niedere Chlorkohlenwasserstoffe verwendet werden.

Verfahrensstufe D-1

Aus den in Stufe C-1 erhaltenen Verbindungen der allgemeinen Formel XI lassen sich durch intramolekulare γ-Lactonbildung unter Abspaltung der Alkohole $R^7OH$, worin $R^7$ die vorgenannten Bedeutungen besitzt, die threo-4-Alkoxy-5-(phenylhydroxymethyl)-2(5H)-furanone der allgemeinen Formel XII, worin $R^0$, $R^1$, $R^3$ und $R^4$ die genannten Bedeutungen besitzen, herstellen. In Abhängigkeit von den Reaktionsbedingungen und der Art des Restes $R^7$, kann die Lactonbildung schon während der Reaktion in Stufe C-1 oder bei der Aufarbeitung des Reaktionsgemisches der Stufe C-1 ganz oder teilweise vor sich gehen. in schwach saurem Milieu schließt sich der Lactonring unter Eliminierung des Alkohols $R^7OH$ besonders leicht.

Verfahrensstufe C-2

Anstelle der Ester der allgemeinen Formel IX können auch die Säuren der allgemeinen Formel VIII direkt der durch Osmiumtetroxid katalysierten cis-Dihydroxylierung unterworfen werden. Die Reaktion führt dann direkt zu den 4.5(threo)-Dihydroxy-2(E)-pentensäure-Derivaten der allgemeinen Formel X, worin $R^0$, $R^1$, $R^3$ und $R^4$ die genannten Bedeutungen besitzen. Als Oxidationsmittel können die in Stufe C-1 beschriebenen Verbindungen verwendet werden.

Auch in diesem Falle wird die Oxidationsreaktion vorzugsweise mit Alkylhydroperoxid in Gegenwart quartärer Ammoniumbasen, beispielsweise in Gegenwart von Tetraethyl- oder Tetrabutylammoniumhydroxid, durchgeführt; die quartären Ammoniumbasen erhöhen die Löslichkeit der an sich schwer löslichen Pentadiensäuren der allgemeinen Formel VIII in wäßrigem Medium und verstärken die katalytische Wirkung des Osmiumtetroxids.

Ebenso wie in Stufe C-1 werden auch in Stufe C-2 stets die 4,5(threo)-Dihydroxy-Verbindungen stereoselektiv erhalten.

Verfahrensstufe D-2

Analog zur γ-Lactonbildung in Stufe D-1 findet der intramolekulare Ringschluß der in Stufe C-2 erhaltenen 4,5(threo)-Dihydroxysäuren der allgemeinen Formel X unter Wasserabspaltung zu den entsprechenden 2(5H)-Furanon-Derivaten der allgemeinen Formel XII mindestens teilweise schon während des Ablaufs der Oxidationsreaktion in Stufe C-2 statt, insbesondere jedoch dann, wenn due Aufarbeitung des Reaktionsgemischs der Stufe C-2 in neutralem oder saurem Milieu erfolgt.

Verfahrensstufe E

Zur Herstellung derjenigen erfindungsgemäßen Verbindungen der allgemeinen Formel I, worin n = 0 und $R^2 \neq H$ ist, werden die in Stufe D-1 oder D-2 erhaltenen threo-4-Alkoxy-5-phenylhydroxymethyl-

2(5H)-furanone der allgemeinen Formel XII, worin $R^0$, $R^1$, $R^3$ und $R^4$ die genannten Bedeutungen besitzen, in die entsprechend substituierten $R^2$-Derivate der allgemeinen Formel XIII, die Ether oder Acetale darstellen, in an sich bekannter Weise umgesetzt.

a) Ether-Derivate der allgemeinen Formel XIII

Zur Herstellung der Ether, in denen $R^2$ eine Alkylgruppe mit 1 bis 3 C-Atomen ist, werden die Verbindungen der allgemeinen Formel XII in einem niederen Alkanon, vorzugsweise in Aceton oder Butanon, bei einer Temperatur zwischen etwa 50 und 100°C bzw. unter Rückfluß mit einem $C_1$- bis $C_3$-Alkyljodid, vorzugsweise mit Methyljodid, in Gegenwart einer mindestens äquimolaren Menge an Silber(I)oxid umgesetzt.

b) Acetal-Derivate der allgemeinen Formel XIII

Zur Herstellung der Acetale, in denen $R^2$ den Rest

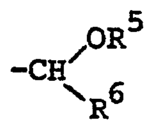

bedeutet, wobei $R^5$ eine Niederalkylgruppe mit 1 bis 5 C-Atomen, die Ethoxyethyl- oder Methoxyethylgruppe und $R^6$ ein Wasserstoffatom, eine Niederalkylgruppe mit 1 bis 5 C-Atomen oder die Methoxymethylgruppe bedeuten, werden die Verbindungen der allgemeinen Formel XII mit einer Verbindung der allgemeinen Formel XIV

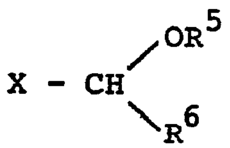

(XIV)

worin $R^5$ und $R^6$ die genannten Bedeutungen besitzen und X einen Methoxy- oder Ethoxy-Rest bedeutet, in Gegenwart einer katalytischen Menge p-Toluolsulfonsäure und in Gegenwart von 0,1 bis 1 Äquivalenten, bezogen auf die Menge an XII, Lithiumbromid umgesetzt. Diese Methode ist im Prinzip bei J.-L. Gras et al., Synthesis 1985, S. 74, beschrieben.

Bevorzugte Acetal-Derivate der allgemeinen Formel XIII sind diejenigen, in denen $R^2$ Methoxymethyl, Ethoxymethyl, Methoxyethoxymethyl, α-Ethoxypropyl oder α,β-Dimethoxyethyl bedeutet.

Die zur Herstellung der Ether und Acetale in Stufe E beschriebenen Verfahren stellen nur beispielhafte Methoden dar; selbstverständlich kann hierfür auch jede andere, dem Fachmann geläufige Methode der Veretherung oder Acetalisierung angewandt werden.

Zur Herstellung derjenigen erfindungsgemäßen Verbindungen der allgemeinen Formel I, worin n = 0, 1 oder 2 ist, wird das von A. Pelter et al. in Tetrahedron Lett. 1979, S. 1627-1630, beschriebene Verfahren analog angewandt. Hierzu werden die 4-Alkoxy-5(2H)-furanone, die, abgeleitet von der Tetronsäure, auch als Alkyltetronate bezeichnet werden können und der allgemeinen Formel XVII entsprechen, worin $R^0$ und $R^1$ die oben genannten Bedeutungen besitzen, mittels geeigneter lithiumorganischer Verbindungen in die Lithium-Derivate der allgemeinen Formel XVIII übergeführt:

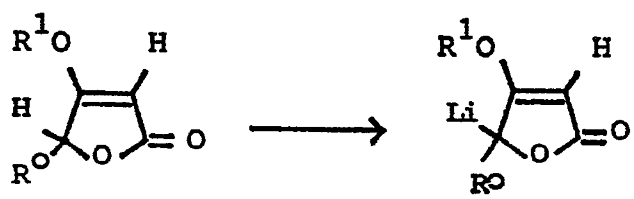

XVII XVIII

welche mit den Aldehyden der allgemeinen Formel XIX

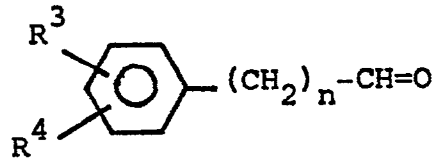

(XIX)

worin $R^3$ und $R^4$ die oben genannten Bedeutungen besitzen und n = 0, 1 oder 2 ist, zu den Verbindungen der allgemeinen Formel I umgesetzt werden, worin n, $R^0$, $R^1$, $R^3$ und $R^4$ die zuvor genannten Bedeutungen besitzen und $R^2$ = H ist.

Bei diesem Analogieverfahren entstehen stets Gemische der threo- und erythro-Diastereomeren, weshalb die threo-Verbindungen anschließend in an sich bekannter, aufwendiger Weise, beispielsweise durch fraktionierte Kristallisation oder chromatographische Verfahren, isoliert werden müssen. Man erhält dann die threo-Verbindungen der allgemeinen Formel I mir $R^2$ = H, die gegebenenfalls anschließend gemäß Stufe E einer Veretherung oder Acetalisierung unterworfen werden können.

Die Alkyltetronate der allgemeinen Formel XVII sind entweder literaturbekannt oder können nach bekannten Verfahren hergestellt werden (vgl. z.B. EP-PS 10 288; J. Org. Chem. 49, 927-928 (1984); Tetrahedron 35, 2181-2185 (1979); Tetrahedron 34, 1453-1455 (1978); Synth. Commun. 10, 805-811 (1980); Angew. Chem. 94, 651-652 (1982)).

Als weitere Methode zur Herstellung der bei diesem Verfahren als Ausgangsmaterial eingesetzten Alkyltetronate der allgemeinen Formel XVII wurde die basenkatalysierte Umalkoxylierung von 4-Methoxy-2(5H)-furanonen gefunden. Hierzu werden die Methoxyfuranone der allgemeinen Formel XVII mit $R^1$ = $CH_3$ mit überschüssigen Niederalkanolen $R^1OH$ mit $R^1$ = $C_2$- bis $C_5$-Niederalkyl unter Zusatz katalytischer Mengen des entsprechenden Alkalialkoholats $R^1$ ONa oder $R^1$ OK in der Siedehitze umgesetzt, wobei unter Abspaltung von Methanol die gewünschten 4-Alkoxy-2(5H)-furanone der allgemeinen Formel XVII entstehen, bei denen $R^1$ eine Niederalkylgruppe mit 2 bis 5 C-Atomen bedeutet.

Gegenstand der Erfindung sind ferner Arzneimittel, die gegebenenfalls zusammen mit einem pharmazeutisch inerten Excipiens eine oder mehrere der Verbindungen der allgemeinen Formel I, einschließlich derjenigen, worin $R^2$ = H oder $CH_3$ bedeutet, wenn n = 0 oder 2, $R^0$ = H, $R^1$ = $CH_3$, $R^3$ = H und $R^4$ = H ist, enthalten.

Diese Arzneimittel und pharmazeutischen Zubereitungen können als Antikonvulsiva in der Human- und Veterinärmedizin und als Antiepileptika in der Humanmedizin verwendet werden. Die wirksame Dosis, in der die Verbindungen verabreicht werden können, liegt sowohl in der Humanmedizin als auch in der Veterinärmedizin zwischen etwa 0,05 und 3 mg/kg. Es können 5 - 200 mg der Wirksubstanz ein- oder mehrmals täglich p.o. appliziert werden.

Als pharmakologisch inerte, übliche Träger- und Zusatzstoffe können beispielsweise Wasser, pflanzliche Öle, Polyethylenglykole, Glycerinester, Gelatine, Kohlenhydrate wie Lactose oder Stärke, Magnesiumstearat, Talk, Vaseline®, Konservierungsmittel, Stabilisatoren, Gleitmittel, netzmittel, Emulgatoren, physiologisch unbedenkliche Salze, Puffersubstanzen, Farbstoffe, Geschmacksstoffe und Aromastoffe verwendet werden. Die Auswahl des pharmakologisch inerten Excipiens hängt davon ab, ob die Wirkstoffe enteral, parenteral oder topikal appliziert werden sollen. Die neuen und bekannten Verbindungen der allgemeinen Formel I können auch im Gemisch mit anderen Wirkstoffen, beispielsweise Vitaminen oder anderen bekannten Antikonvulsiva oder Antiepileptika, verabreicht werden.

Jede der in den nachfolgenden Beispielen genannten erfindungsgemäßen Verbindungen und Zwischenprodukte stellt ein zur Herstellung pharmazeutischer Zubereitungen besonders geeignetes Mittel dar.

In den folgenden Beispielen werden folgende Abkürzungen verwendet:
Schmp. = Schmelzpunkt (unkorrigiert)
Kp. = Siedepunkt (unkorrigiert)
(Z) = Zersetzung
Temperaturen sind in °C angegeben
Drücke sind in bar (mbar) angegeben, wobei 1 bar (mbar) $\neq 10^5$ ($10^2$) Pa.

Die 300 MHz-$^1$H- und 75,46 MHz-$^{13}$C-Kernresonanzspektren wurden auf dem NMR-Spektrometer WH 300 der Firma Bruker aufgenommen. Als Lösungsmittel wurde hierfür, sofern nicht anders angegeben, DMSO-$D_6$ verwendet. Die chemischen Verschiebungen δ sind relativ zum Tetramethylsilan-Signal (innerer Standard) in ppm angegeben.

Beispiel Nr. 1

Ethyl-3-methoxy-2(E)-butenoat:

Das Gemisch aus 520 g (4 Mol) Ethylacetoacetat, 400 ml Methanol und 2 ml 36%iger Salzsäure (oder 1 ml konz. Schwefelsäure) wird auf 50°C erwärmt. Unter Rühren tropft man 425 g (4 Mol) Trimethylorthoformiat so zu, daß die Mischung bei ca. 50°C gehalten wird. Anschliessend destilliert man gebildetes Methylformiat und überschüssiges Methanol ab und fraktioniert den Rückstand über eine Vigreux-Kolonne. Zwischen 184° und 186°C destillieren 548 g (3.8 Mol) reines Ethyl-3-methoxy-2(E)-butenoat über. Ausbeute: 95%.

Beispiel Nr. 2

Ethyl-3-ethoxy-2(E)-butenoat:

Herstellung analog Beispiel Nr. 1 durch Umsetzung von Ethylacetoacetat mit Triethylorthoformiat in Ethanol mit Salzsäure als Katalysator. Ausbeute: 84.5%.
Kp = 191°- 195°C. Schmp. = 31°- 33°C.

Beispiel Nr. 3

Methyl-3-methoxy-2(E)-pentenoat:

Herstellung analog Beispiel Nr. 1 durch Umsetzung von Methyl-3-oxopentanoat mit Trimethylorthoformiat in Methanol und Schwefelsäure als Katalysator.
Ausbeute: 87.7%. Kp = 76° - 78°C (20 mbar).

Beispiel Nr. 4

Ethyl-3-methoxy-2(E)-hexenoat:

Herstellung analog Beispiel Nr. 1 durch Umsetzung von Ethyl-3-oxohexanoat mit Trimethylorthoformiat in Methanol und Schwefelsäure als Katalysator.
Ausbeute: 95.8%. Kp = 87°- 89°C (20 mbar).

Beispiel Nr. 5

Ethyl-3-methoxy-5-methyl-2(E)-hexenoat:

Herstellung analog Beispiel Nr. 1 durch Umsetzung von Ethyl-5-methyl-3-oxohexanoat mit Trimethylorthoformiat in Methanol und Schwefelsäure als Katalysator.
Ausbeute: ca. 50% Kp = 91° - 97°C (16-19 mbar)

Beispiel Nr. 6

3-Methoxy-5-phenyl-2(E),4(E)-pentadiensäure:

Methode a:

Unter Rühren und Stickstoffatmosphäre werden zu der Mischung aus 53 g (0.5 Mol) Benzaldehyd, 100 ml Dimethylsulfoxid und 72 g (0.5 Mol) Ethyl-3-methoxy-2(E)-butenoat 11.6 g (0.05 Mol) Benzyltriethylammoniumchlorid zugefügt und danach die Lösung von 33.6 g (0.6 Mol) Kaliumhydroxid in 35 ml Wasser zugetropft. Man erwärmt ca. 16 Stdn. lang auf 110°C, evaporiert die Lösung, löst den Rückstand in 400 ml Wasser und extrahiert Ausgangs- und Nebenprodukte mit 100 ml Dichlormethan. Aus der wäßrigen Phase fällt nach Ansäuern mit 70 ml 10M-Salzsäure das Rohprodukt aus, das nach Absaugen, säurefrei Waschen mit Wasser, Umkristallisation aus Ethanol und Trocknen bei 100°C im Vakuum 34.9 g (0.171 Mol) Reinprodukt mit Schmp. = 154 - 155°C ergibt.
Ausbeute 34.2%.
Literatur Schmp. = 157,5 - 158°C [ E.E. Smissman und A.N. Voldeng, J. Org. Chem. 29, 3161 (1964)].
Analyse: $C_{12}H_{12}O_3$ (204.23)
Ber.: C (70.57), H (5.92)
Gef.: C (70.49), H (6.16)

Methode b:

Unter Rühren und Stickstoffatmosphäre werden zu der Lösung von 3785 g (26.25 Mol) Ethyl-3-methoxy-2(E)-butenoat in 6 Litern Dimethylsulfoxid nacheinander 2653 g (25 Mol) Benzaldehyd und 926 ml (2.5 Mol) 40%iges wäßriges Tetraethylammoniumhydroxid zugetropft. Nach Erhitzen auf 100°C tropft man die Lösung von 1470 g (26.25 Mol) Kaliumhdyroxid in 1500 ml Wasser zu und rührt 4 Stdn. bei 100°C. Nach Abkühlen auf ca. 20°C gießt man die Mischung in 50 Liter Wasser und extrahiert Verunreinigungen mit 10 Litern Dichlormethan. Die Wasserphase wird unter kräftigem Rühren mit ca. 3 Litern 33%iger

Salzsäure auf pH = 2 angesäuert und das ausgefällte Rohprodukt über einen Druckfilter abfiltriert, mit Wasser chloridfrei gewaschen und mit Stickstoff trocken geblasen. Der Filterkuchen wird in 6 Litern Ethanol suspendiert, erneut filtriert und nach Trockenblasen im Vakuum bis zur Endtemperatur von 85°C getrocknet. Man erhält 3071 g (15.04 Mol) Reinprodukt mit Schmp. 159°- 160°C . Ausbeute: 60.1%.

Beispiel Nr. 7

5-(2-Chlorphenyl)-3-methoxy-2(E),4(E)-pentadiensäure:

3785 g (26.25 Mol) Ethyl-3-methoxy-2(E)-butenoat werden analog Beispiel Nr. 6 b in 6 Litern Dimethylsulfoxid mit 3515 g (25 Mol) 2-Chlorbenzaldehyd, 926 ml (2.5 Mol) 40%igem Tetraethylammoniumhydroxid und 2940g(26.25 Mol) 50%iger Kalilauge umgesetzt und 4 Stdn. bei 100°C nachgerührt. Nach dem Abkühlen vedünnt man mit 10 Litern Wasser und extrahiert Nebenprodukte mit 10 Litern Dichlormethan. Die wäßrige Phase wird in die Mischung aus 3 Litern 33%iger Salzsäure und 50 Litern Waser eingerührt, wonach der pH-Wert 3,5 bis 4 beträgt. Das ausgefällte Rohprodukt wird nach Filtrieren, Waschen mit Wasser bis zur Chloridfreiheit und Trockenblasen in 20 Litern Ethanol suspendiert erneut abgesaugt, trockengeblasen und bis zur Endtemperatur von 85°C bei 20 mbar getrocknet. Man erhält 5045 g (21.14 Mol) Reinprodukt mit Schmp. 202°C. Ausbeute: 84.55%
Analyse: $C_{12}H_{11}ClO_3$ (238.67)
Ber.: C (60.39), H (4.65), Cl (14.85)
Gef.: C (60.33), H (4.85), Cl (14.72)
300 MHz-$^1$H-NMR: 11.8 - 12.2 (1 H, br.m, COOH), 8.08 (1 H, d, $J_{5/4}$ = 16 Hz, H-5), 7.54 (1 H, d, $J_{4/5}$ = 16 Hz, H-4), 5.29 ( 1 H,s, H-2), 3.785 (3 H, s, $OCH_3$), 7.35 - 7.75 (4 H, m, aromat. Protonen).

Analog zu den in Beispielen Nr. 6 a, b und 7 verwendeten Synthese-Methoden werden durch Kondensation von Benzaldehyd bzw. substituierten Benzaldehyden mit den in Beispielen Nr. 1 bis 5 beschriebenen 3-Alkoxy-2(E)-alkenoaten folgende 3-Alkoxy-5-(subst.)phenyl-2(E),4(E)-pentadiensäuren hergestellt: siehe Tabelle 1.
In den Tabellen 2 und 3 sind die analytischen und $^1$H-NMR-spektroskopischen Daten aufgelistet.

Tabelle 1: 3-Alkoxy-5-phenylpentadiensäuren

| Beispiel Nr. | Bezeichnung | Ausbeute [%] +) | Schmp. [°C] (umkristallisiert aus) |
|---|---|---|---|
| 8 | 5-(2-Chlorphenyl)-3-ethoxy-2(E),4(E)-pentadiensäure | 41 | 179-181 (Ethanol) |
| 9 | 5-(2-Chlorphenyl)-3-methoxy-4-methyl-2(E),4(E)-pentadiensäure | 24 | 126-128 (Methanol) |
| 10 | 5-(2-Chlorphenyl)-4-ethyl-3-methoxy-2(E),4(E)-pentadiensäure | 8.3 | 131-132 (Ethanol) |
| 11 | 5-(2-Chlorphenyl)-4-isopropyl-3-methoxy-2(E),4(E)-pentadiensäure | | |
| 12 | 5-(4-Chlorphenyl)-3-methoxy-2(E),4(E)-pentadiensäure | 36 | 200 (Ethanol) |
| 13 | 5-(2-Bromphenyl)-3-methoxy-2(E),4(E)-pentadiensäure | 71 | 206-207 (Ethanol) |
| 14 | 5-(3-Bromphenyl)-3-methoxy-2(E),4(E)-pentadiensäure | 14 | 161-163 (Ethanol) |
| 15 | 5-(2-Fluorphenyl)-3-methoxy-2(E),4(E)-pentadiensäure | 66 | 188-190 (Ethanol) |
| 16 | 5-(2-Trifluormethylphenyl)-3-methoxy-2(E),4(E)-pentadiensäure | 15 | 173-174 (Ether) |
| 17 | 5-(3-Trifluormethylphenyl)-3-methoxy-2(E),4(E)-pentadiensäure | 41 | 152-153 (Ethanol) |
| 18 | 5-(4-Trifluormethylphenyl)-3-methoxy-2(E),4(E)-pentadiensäure | 32 | 181-183 (Methanol) |
| 19 | 3-Methoxy-5-(2-nitrophenyl)-2(E),4(E)-pentadiensäure | | 209-211 (Ethanol) |

Fortsetzung Tabelle 1

| Beispiel Nr. | Bezeichnung | Ausbeute [%]+) | Schmp.[°C] (umkristallisiert aus) |
|---|---|---|---|
| 20 | 3-Methoxy-5-(3-nitrophenyl)-2(E),4(E)-pentadiensäure | 9 | 198-199 |
| 21 | 3-Methoxy-5-(2,5-dimethylphenyl)-2(E),4(E)-pentadiensäure | 49 | 161-162 (Ether) |
| 22 | 5-(2-Chlor-5-methylphenyl)-3-methoxy-2(E),4(E)-pentadiensäure | 48 | 179-181 (Methanol) |
| 23 | 5-(2,3-Dichlorphenyl)-3-methoxy-2(E),4(E)-pentadiensäure | 10 | 215-216 (Ethanol) |
| 24 | 5-(2,4-Dichlorphenyl)-3-methoxy-2(E),4(E)-pentadiensäure | 73 | 196-197 (Ethanol) |
| 25 | 5-(2,4-Dichlorphenyl)-3-ethoxy-2(E),4(E)-pentadiensäure | 50 | 198-200 (Ethanol) |
| 26 | 5-(2,5-Dichlorphenyl)-3-methoxy-2(E),4(E)-pentadiensäure | 55 | 198-199 (Ethanol) |
| 27 | 5-(2,5-Dichlorphenyl)-3-ethoxy-2(E),4(E)-pentadiensäure | 49 | 213-215 (Ethanol) |
| 28 | 5-(3,4-Dichlorphenyl)-3-methoxy-2(E),4(E)-pentadiensäure | 46 | 188-190 (Ethanol) |
| 29 | 5-(3,5-Dichlorphenyl)-3-methoxy-2(E),4(E)-pentadiensäure | 38 | 211-213 (2-Propanol) |
| 30 | 5-[3,5-Bis-(trifluormethyl)-phenyl]-3-methoxy-2(E),4(E)-pentadiensäure | 6 | 217-219 (Chloroform) |
| 31 | 5-(2-Chlor-5-trifluormethylphenyl)-3-methoxy-2(E),4(E)-pentadiensäure | 40 | 188-192 (Ethanol) |
| 32 | 5-(4-Chlor-2-trifluormethylphenyl)-3-methoxy-2(E),4(E)-pentadiensäure | 22 | 190-193 (Ethanol) |

Fortsetzung Tabelle 1

| Beispiel Nr. | Bezeichnung | Ausbeute [%] | Schmp.[°C] (umkristallisiert aus) |
|---|---|---|---|
| 33 | 5-(4-Brom-2-chlorphenyl)-3-methoxy-2(E),4(E)-pentadiensäure | 66 | 192-194 (Aceton) |

+)Bei Ausbeuten unter 50 % sind Reaktions-, Aufarbeitungs- und Umkristallisationsbedingungen noch nicht optimiert.

Tabelle 2: Elementaranalysen

| Beispiel Nr. | Summenformel / Molmasse | % Ber. C / % Gef. C | H / H | Sonstige / " |
|---|---|---|---|---|
| 8 | $C_{13}H_{13}ClO_3$ | 61.79 | 5.18 | Cl (14.03) |
| | 252.69 | 61.91 | 5.31 | 14.3 |
| 9 | $C_{13}H_{13}ClO_3$ | 61.79 | 5.18 | Cl (14.03) |
| | 252.69 | 61.46 | 5.15 | 13.7 |
| 10 | $C_{14}H_{15}ClO_3$ | 63.05 | 5.67 | Cl (13.29) |
| | 266.73 | 63.16 | 6.00 | 13.5 |
| 12 | $C_{12}H_{11}ClO_3$ | 60.39 | 4.65 | Cl(14.85) |
| | 238.67 | 59.80 | 4.42 | 15.5 |
| 13 | $C_{12}H_{11}BrO_3$ | 50.91 | 3.92 | Br (28.22) |
| | 283.13 | 51.01 | 3.38 | 28.0 |
| 14 | $C_{12}H_{11}BrO_3$ | 50.91 | 3.92 | Br (28.22) |
| | 283.13 | 50.06 | 3.90 | 30.5 |
| 15 | $C_{12}H_{11}FO_3$ | 64.86 | 4.99 | F ( 8.55) |
| | 222.22 | 64.56 | 4.88 | 7.9 |
| 16 | $C_{13}H_{11}F_3O_3$ | 57.36 | 4.07 | F (20.94) |
| | 272.23 | 57.40 | 4.13 | 20.6 |
| 17 | $C_{13}H_{11}F_3O_3$ | 57.36 | 4.07 | F (20.94) |
| | 272.23 | 56.99 | 3.90 | |
| 18 | $C_{13}H_{11}F_3O_3$ | 57.36 | 4.07 | F (20.94 ) |
| | 272.23 | 57.15 | 3.91 | |
| 19 | $C_{12}H_{11}NO_5$ | 57.83 | 4.45 | N ( 5.62) |
| | 249.23 | 57.97 | 4.33 | 5.35 |
| 20 | $C_{12}H_{11}NO_5$ | 57.83 | 4.45 | N ( 5.62) |
| | 249.23 | 58.15 | 4.66 | 5.73 |
| 21 | $C_{14}H_{16}O_3$ | 72.39 | 6.94 | |
| | 232.29 | 72.27 | 7.32 | |
| 22 | $C_{13}H_{13}ClO_3$ | 61.79 | 5.18 | Cl (14.03) |
| | 252.69 | 62.11 | 5.15 | 14.5 |
| 23 | $C_{12}H_{10}Cl_2O_3$ | 52.77 | 3.69 | Cl (25.96) |
| | 273.12 | 52.78 | 3.65 | 25.7 |
| 24 | $C_{12}H_{10}Cl_2O_3$ | 52.77 | 3.69 | Cl (25.96) |
| | 273.12 | 52.81 | 3.76 | 26.2 |
| 25 | $C_{13}H_{12}Cl_2O_3$ | 54.38 | 4.21 | Cl (24.69) |
| | 287.15 | 54.40 | 4.19 | 24.9 |

Fortsetzung Tabelle 2

| Beispiel Nr. | Summenformel / Molmasse | % Ber. C / % Gef. C | H / H | Sonstige / " |
|---|---|---|---|---|
| 26 | $C_{12}H_{10}Cl_2O_3$ | 52.77 | 3.69 | Cl (25.96) |
| | 273.12 | 52.90 | 3.75 | 26.2 |
| 27 | $C_{13}H_{12}Cl_2O_3$ | 54.38 | 4.21 | Cl (24.69) |
| | 287.15 | 53.98 | 3.98 | 24.9 |
| 28 | $C_{12}H_{10}Cl_2O_3$ | 52.77 | 3.69 | Cl (25.96) |
| | 273.12 | 52.95 | 3.84 | 26.2 |
| 29 | $C_{12}H_{10}Cl_2O_3$ | 52.77 | 3.69 | Cl (25.96) |
| | 273.12 | 52.64 | 3.87 | 26.2 |
| 30 | $C_{14}H_{10}F_6O_3$ | 49.43 | 2.96 | F (33.50) |
| | 340.23 | 48.90 | 2.88 | |
| 31 | $C_{13}H_{10}ClF_3O_3$ | 50.91 | 3.29 | Cl (11.56) |
| | 306.67 | 49.96 | 3.11 | 11.2 |
| 32 | $C_{13}H_{10}ClF_3O_3$ | 50.91 | 3.29 | Cl (11.56) |
| | 306.67 | 50.74 | 2.99 | 11.5 |
| 33 | $C_{12}H_{10}BrClO_3$ | 45.39 | 3.17 | Cl (11.16) |
| | 317.57 | 45.35 | 3.00 | 10.8 |

Tabelle 3: 300 MHz - $^1H$ - NMR Spektroskopie.

| Beispiel Nr. | δ - Werte [ppm] für (breit) -COOH | H-2(s) | 3-OCH$_3$(s) | H-4(d) | H-5(d) | $J_{H-4/H-5}$ [Hz] |
|---|---|---|---|---|---|---|
| 8 | 11.96 | 5.25 | (3-OCH$_2$-) 3.99(q) | 7.55 | 8.08 | 16.2 |
| 10 | 11.46 | 5.17 | 3.7 | (-CH$_2$-) 2.34(q) | 6.44 | - |
| 12 | 11.7 | 5.22 | 3.73 | 7.20 | 8.01 | 16.1 |
| 14 | 11.95 | 5.23 | 3.75 | 7.23 | 8.03 | 16.4 |
| 15 | 11.75 | 5.24 | 3.74 | 7.30 | 8.11 | 16.6 |
| 16 | 11.8 | 5.29 | 3.76 | 7.5 | 8.05 | 16.1 |
| 17 | 11.78 | 5.25 | 3.75 | 7.30 | 8.09 | 16.6 |
| 18 | 11.8 | 5.26 | 3.75 | 7.28 | 8.12 | 16.6 |
| 19 | 11.99 | 5.3 | 3.77 | 7.51 | 8.02 | 16.2 |
| 20 | 12.02 | 5.29 | 3.77 | 7.39 | 8.16 | 16.4 |
| 21 | 11.65 | 5.21 | 3.74 | 7.42 | 7.90 | 16.1 |
| 22 | 11.5 | 5.26 | 3.77 | 7.49 | 8.01 | 16.4 |
| 23 | 12 | 5.31 | 3.78 | 7.52 | 8.06 | 16.4 |
| 24 | 12.0 | 5.29 | 3.77 | 7.46 | 8.07 | 15.8 |
| 25 | 12 | 5.26 | (3-OCH$_2$-) 3.99(q) | 7.47 | 8.06 | 16.4 |
| 26 | 12.04 | 5.31 | 3.78 | 7.43 | 8.05 | 16.2 |
| 27 | | 5.27 | (3-OCH$_2$-) 3.99(q) | 7.44 | 8.04 | 15.8 |
| 28 | 12 | 5.25 | 3.75 | 7.22 | 8.03 | 16.4 |
| 29 | 11.8 | 5.27 | 3.74 | 7.19 | 8.11 | 16.1 |
| 31 | 12 | 5.32 | 3.78 | 7.50 | 8.11 | 16.4 |
| 32 | 11.9 | 5.31 | 3.77 | 7.42 | 8.06 | 16.4 |
| 33 | 11.9 | 5.29 | 3.78 | 7.44 | 8.06 | 16.4 |

Beispiel Nr. 34

Benzyl-5-(2-chlorphenyl)-3-methoxy-2(E),4(E)-pentadienoat:

Zu der Mischung aus 48 g (0.2 Mol) 5-(2-Chlorphenyl)-3-methoxy-2(E),4(E)-pentadiensäure, 600 ml Aceton und 60 g (0,43 Mol) Kaliumcarbonat tropft man unter Rühren 34.2 g (0.2 Mol) Benzylbromid und erhitzt 16 Stdn. zum Rückfluß. Nach Abfiltrieren anorganischer Rückstände, Evaporieren des Filtrats und Umkristallisation aus tert. Butylmethylether erhält man 56.1 g (0.17 Mo reinen Benzylester mit Schmp. 83 - 86°C. Ausbeute: 85 %.
Analyse: $C_{19}H_{17}ClO_3$ (328.80)
Ber.: C (69.41), H (5.21), Cl (10.78)
Gef.: C (69.42), H (5.08), Cl (10.9 ).

Beispiel Nr. 35

Ethyl-5-(2-chlorphenyl)-3-methoxy-2(E),4(E)-pentadienoat:

a) Die Mischung aus 31 g (0.13 Mol) 5-(2-Chlorphenyl)-3-methoxy-2(E),4(E)-pentadiensäure, 400 ml Aceton, 36g(0.26 Mol) Kaliumcarbonat und 2o.8 g (0.13 Mol) Jodethan wird 18 Stdn. unter Rückfluß zum Sieden erhitzt. Nach dem Abkühlen, Filtrieren und Evaporieren des Filtrats nimmt man das ölige Rohprodukt in 100 ml n-Pentan auf, filtriert unlösliches Kaliumjodid ab, evaporiert das Filtrat und trocknet bei 45°C und 20 mbar nach. Man erhält 33.3 g (0.125 Mol) reinen Ethylester mit Schmp. 49 - 51°C, Ausbeute: 96%
Analyse: $C_{14}H_{15}ClO_3$ (266.73)
Ber.: C (63.05), H (5.67), Cl(13.29)
Gef.: C (63.37), H (5.69), Cl(13.3)

b) Unter Rühren tropft man zu dem Gemisch aus 35 Litern Butanon, 2386 g (10 Mol) 5-(2-Chlorphenyl)-3-methoxy-2(E),4(E)-pentadiensäure, 2073g(15 Mol) Kaliumcarbonat und 16.6 g (0.1 Mol) Kaliumjodid bei 56°C 1635 g (15 Mol) Bromethan und rührt 24 Stdn. bei 56°C nach. Nach dem Abkühlen und Abfiltrieren anorganischer Bestandteile wäscht man das Filtrat mit 2 mal 10 Litern Wasser und evaporiert die Butanonphase. Man erhält 2348 g (8.80 Mol) Ethylester, der beim Erkalten kristallisiert. Dünnschichtchromatographie und Infrarotspektrum zeigen die Identität mit dem zuvor beschriebenen Produkt (35 a) an. Schmp. 45°C. Ausbeute: 88%

Beispiel Nr. 36

Methyl-5-(2-chlorphenyl)-3-methoxy-2(E),4(E)-pentadienoat:

Das Gemisch aus 23.9 g (0.1 Mol) 5-(2-Chlorphenyl)-3-methoxy-2(E),4(E)-pentadiensäure, 500 ml Aceton und 30.4 g (0.22 Mol) Kaliumcarbonat wird unter Rühren mit 9.5 ml (0.1 Mol) Dimethylsulfat versetzt und 4 Stdn. lang zum Rückfluß erhitzt. Nach dem Abkühlen und Filtrieren evaporiert man das Filtrat, löst das Rohprodukt in 100 ml Chloroform, wäscht mit 2 mal 30 ml Wasser, evaporiert die Chloroformphase und kristallisiert aus Isopropanol/Wasser um. Nach Trocknen bei 50°C/20 mbar erhält man 22.18 g (87.8 mmol) reinen Methylester mit Schmp. 50 - 52°C. Ausbeute: 87.8 %
Analyse: $C_{13}H_{13}ClO_3$ (252.69)
Ber.: C (61.79), H (5.18), Cl(14.03)
Gef.: C (61.30), H (5.07), Cl(13.7)

Analog zu den in Beispielen Nr. 34 bis 36 beschriebenen Methoden wurden die in Tabelle 4 aufgeführten 3-Alkoxy-5-phenyl-2(E),4(E)-pentadiensäureester hergestellt. Ergebnisse der Elementaranalysen sind in Tabelle 5 zusammengefaßt.

Tabelle 4: 3-Alkoxy-5-phenylpentadienoate

| Beispiel Nr. | Bezeichnung | Ausbeute [%] | Schmp.[°C] (umkristallisiert aus:) |
|---|---|---|---|
| 37 | Isopropyl-5-(2-chlorphenyl)-3-methoxy-2(E),4(E)-pentadienoat | 95 | 55-58 (Pentan) |
| 38 | sec.Butyl-5-(2-chlorphenyl)-3-methoxy-2(E),4(E)pentadienoat | 85 | 41-42 (Isopropanol/Wasser) |
| 39 | tert.Butyl-5-(2-chlorphenyl)-3-methoxy-2(E),4(E)-pentadienoat | 30 | Oel (evaporiert) |
| 40 | Ethyl-3-methoxy-5-phenyl-2(E),4(E)-pentadienoat | 96 | 34 (evaporiert) |
| 41 | Ethyl-5-(2-chlorphenyl)-3-ethoxy-2(E),4(E)-pentadienoat | 85 | 79-81 (Methanol) |
| 42 | Ethyl-5-(4-chlorphenyl)-3-methoxy-2(E),4(E)-pentadienoat | 94 | 52-53 (Isopropanol/Pentan) |
| 43 | Ethyl-5-(2-Bromphenyl)-3-methoxy-2(E),4(E)-pentadienoat | 98 | 45-46 (Pentan) |
| 44 | Ethyl-5-(2-fluorphenyl)-3-methoxy-2(E),4(E)-pentadienoat | 99 | 46-48 (Isopropanol) |
| 45 | Ethyl-5-(2-trifluormethylphenyl)-3-methoxy-2(E),4(E)-pentadienoat | 99 | 75 (Ethanol) |
| 46 | Ethyl-5-(3-trifluormethylphenyl)-3-methoxy-2(E),4(E)-pentadienoat | 92 | 46-47 (Ethanol) |
| 47 | Ethyl-5-(4-trifluormethylphenyl)-3-methoxy-2(E),4(E)-pentadienoat | 52 | 42-44 (Ethanol) |

1. Fortsetzung Tabelle 4

| Beispiel Nr. | Bezeichnung | Ausbeute [%] | Schmp. [°C] (umkristallisiert aus:) |
|---|---|---|---|
| 48 | Ethyl-3-methoxy-5-(2.5-dimethylphenyl)-2(E),4(E)-pentadienoat | 94 | 53-54 (Ethanol) |
| 49 | Ethyl-5-(2-chlor-5-methylphenyl)-3-methoxy-2(E),4(E)-pentadienoat | 95 | 76-78 (Ethanol) |
| 50 | Ethyl-5-(2.3-dichlorphenyl)-3-methoxy-2(E),4(E)-pentadienoat | 78 | 110-111 (Ethanol) |
| 51 | Ethyl-5-(2.4-dichlorphenyl)-3-methoxy-2(E),4(E)-pentadienoat | 92 | 81-83 (Isopropanol) |
| 52 | Ethyl-5-(2.4-dichlorphenyl)-3-ethoxy-2(E),4(E)-pentadienoat | 92 | 64-67 (Isopropanol) |
| 53 | Ethyl-5-(2.5-dichlorphenyl)-3-methoxy-2(E),4(E)-pentadienoat | 92 | 97-98 (Ethanol) |
| 54 | Ethyl-5-(2.5-dichlorphenyl)-3-ethoxy-2(E),4(E)-pentadienoat | 64 | 87-90 (Isopropanol) |
| 55 | Ethyl-5-(3.4-dichlorphenyl)-3-methoxy-2(E),4(E)-pentadienoat | 89 | 76-78 (Ethanol) |
| 56 | Ethyl-5-(3.5-dichlorphenyl)-3-methoxy-2(E),4(E)-pentadienoat | 93 | 89-91 (Ethanol) |
| 57 | Ethyl-5-[3.5-bis-(trifluormethyl)-phenyl]-3-methoxy-2(E),4(E)-pentadienoat | 88 | 86-88 (evaporiert) |
| 58 | Ethyl-5-(2-chlor-5-trifluormethylphenyl)-3-methoxy-2(E),4(E)-pentadienoat | 76 | 47-50 (Ethanol) |

2. Fortsetzung Tabelle 4

| Beispiel Nr. | Bezeichnung | Ausbeute [%] | Schmp. [°C] (umkristallisiert aus:) |
|---|---|---|---|
| 59 | Ethyl-5-(4-chlor-2-trifluormethylphenyl)-3-methoxy-2(E),4(E)-pentadienoat | 92 | 79-80 (Ethanol / Wasser) |
| 60 | Ethyl-5-(4-brom-2-chlorphenyl)-3-methoxy-2(E),4(E)-pentadienoat | 91 | 91-93 (Ethanol) |
| 61 | Ethyl-5-(2-chlorphenyl)-3-methoxy-4-methyl-2(E),4(E)-pentadienoat | 98 | Oel (evaporiert) |
| 62 | Ethyl-5-(2-chlorphenyl)-4-ethyl-3-methoxy-2(E),4(E)-pentadienoat | 92 | 49 (Ethanol) |
| 63 | Ethyl-5-(2-chlorphenyl)-4-isopropyl-3-methoxy-2(E),4(E)-pentadienoat | | |

Tabelle 5: Elementaranalysen

| Beispiel Nr. | Summenformel / Molmasse | %Ber. C / %Gef. C | H / H | Sonstige / " |
|---|---|---|---|---|
| 37 | $C_{15}H_{17}ClO_3$ | 64.17 | 6.10 | Cl(12.63) |
| | 280.76 | 64.38 | 6.13 | 12.3 |
| 38 | $C_{16}H_{19}ClO_3$ | 65.19 | 6.50 | Cl(12.03) |
| | 294.78 | 64.88 | 6.68 | 11.7 |
| 40 | $C_{14}H_{16}O_3$ | 72.39 | 6.94 | |
| | 232.29 | 72.85 | 6.78 | |
| 41 | $C_{15}H_{17}ClO_3$ | 64.17 | 6.10 | Cl(12.63) |
| | 280.76 | 64.12 | 6.38 | 13.0 |
| 42 | $C_{14}H_{15}ClO_3$ | 63.05 | 5.67 | Cl(13.29) |
| | 266.73 | 62.99 | 5.98 | 13.5 |
| 43 | $C_{14}H_{15}BrO_3$ | 54.04 | 4.86 | Br(25.86) |
| | 311.19 | 53.70 | 4.83 | 25.0 |
| 44 | $C_{14}H_{15}FO_3$ | 67.19 | 6.04 | F (7.59) |
| | 250.28 | 66.78 | 5.61 | 7.3 |
| 45 | $C_{15}H_{15}F_3O_3$ | 60.00 | 5.04 | F (18.98) |
| | 300.29 | 60.35 | 5.32 | 17.9 |
| 46 | $C_{15}H_{15}F_3O_3$ | 60.00 | 5.04 | F (18.98) |
| | 300.29 | 60.19 | 5.35 | 18.3 |
| 47 | $C_{15}H_{15}F_3O_3$ | 60.00 | 5.04 | F (18.98) |
| | 300.29 | 60.11 | 5.28 | 17.6 |
| 48 | $C_{16}H_{20}O_3$ | 73.82 | 7.74 | |
| | 260.34 | 74.45 | 7.81 | |
| 49 | $C_{15}H_{17}ClO_3$ | 64.17 | 6.10 | Cl(12.63) |
| | 280.76 | 63.90 | 6.03 | 13.0 |
| 50 | $C_{14}H_{14}Cl_2O_3$ | 55.83 | 4.68 | Cl(23.54) |
| | 301.18 | 56.06 | 4.57 | 23.2 |
| 51 | $C_{14}H_{14}Cl_2O_3$ | 55.83 | 4.68 | Cl(23.54) |
| | 301.18 | 55.80 | 4.81 | 23.8 |
| 52 | $C_{15}H_{16}Cl_2O_3$ | 57.16 | 5.12 | Cl(22.50) |
| | 315.21 | 56.85 | 5.08 | 22.7 |
| 53 | $C_{14}H_{14}Cl_2O_3$ | 55.83 | 4.68 | Cl(23.54) |
| | 301.18 | 55.49 | 4.73 | 23.7 |

Fortsetzung Tabelle 5

| Beispiel Nr. | Summenformel / Molmasse | % Ber. C / % Gef. C | H / H | Sonstige / Sonstige |
|---|---|---|---|---|
| 54 | $C_{15}H_{16}Cl_2O_3$ | 57.16 | 5.12 | Cl(22.50) |
| | 315.21 | 56.73 | 5.21 | 21.5 |
| 55 | $C_{14}H_{14}Cl_2O_3$ | 55.83 | 4.68 | Cl(23.54) |
| | 301.18 | 55.52 | 4.57 | 23.7 |
| 56 | $C_{14}H_{14}Cl_2O_3$ | 55.83 | 4.68 | Cl(23.54) |
| | 301.18 | 55.48 | 4.85 | 23.7 |
| 58 | $C_{15}H_{14}ClF_3O_3$ | 53.83 | 4.22 | Cl(10.59) |
| | 334.73 | 53.43 | 4.14 | 11.7 |
| 60 | $C_{14}H_{14}BrClO_3$ | 48.65 | 4.08 | Cl(10.26) |
| | 345.63 | 48.72 | 3.95 | 10.5 |
| 62 | $C_{16}H_{19}ClO_3$ | 65.19 | 6.50 | Cl(12.03) |
| | 294.78 | 66.29 | 6.83 | 12.2 |

Beispiel Nr. 64

threo-5-(2-Chlorphenylhydroxymethyl)-4-methoxy-2(5H)-furanon:

Anhand der Synthese dieser Zielverbindung werden die verschiedenen Ausführungsformen der durch Osmiumtetroxid katalysierten Oxidation der 3-Alkoxy-5-phenyl-2(E)-4(E)-pentadiensäure-Derivate zu den threo-4-Alkoxy-5-phenylhydroxymethyl-2(5H)-furanonen dargestellt (s. Schema 1, Stufen C-1, C-2, D-1 und D-2).

Verfahrensvariante a:

Zu der Mischung aus 48.0 g (201 mmol) 5-(2-Chlorphenyl)-3-methoxy-2(E),4(E)-pentadiensäure, 400 ml Wasser und 400 ml (566 mmol) 20 %igem wäßrigen Tetraethylammoniumhydroxid gibt man unter Rühren und Kühlen auf 0°C 50 ml einer 0.02M-Lösung von Osmiumtetroxid in Aceton oder tert. Butanol und 56 ml (403 mmol) 70%iges wäßriges tert. Butylhydroperoxid. Nach 6 Stdn. Rühren bei 0°C läßt man 8 Tage bei 0-5°C stehen und reduziert danach überschüssiges Hydroperoxid durch Verrühren mit 10%iger wäßriger Natriumsulfit-Lösung. Durch Zugabe von 1M-Schwefelsäure stellt man auf pH=2, wobei nicht umgesetztes Ausgangsprodukt auskristallisiert. Dieses wird abgesaugt (10.1 g≙42.3 mmol). Das Filtrat wird mit 3 mal 100 ml Chloroform extrahiert und der Extrakt mit 50 ml 1M-Salzsäure frei von quartären Ammoniumsalzen gewaschen. Der Extrakt enthält ein Gemisch aus threo-5-(2-Chlorphenyl)-4,5-dihydroxy-3-methoxy-2(E)-pentensäure und threo-5-(2-Chlorphenylhydroxymethyl)-4-methoxy-2(5H)-furanon. Durch Evaporieren vervollständigt man den Ringschluß und erhält nach Umkristallisation aus Ethylacetat 18.12 g (71.2 mmol) reines threo-5-(2-Chlorphenylhydroxymethyl)-4-methoxy-2(5H)-furanon mit Schmp. 149-151°C. Ausbeute bezogen auf umgesetztes Ausgangsprodukt: 45.1%.
Analyse: $C_{12}H_{11}ClO_4$ (254.67)
Ber.: C (56.59), H (4.35), Cl (13.92)
Gef.: C (56.27), H (4.35), Cl (14.0)
300 MHz-$^1$-NMR: 7.3-7.7 (4 H, m, aromatische Protonen),
5.96 ( 1H, d, $J_{OH/H\alpha}$ = 5.5 Hz, α-OH),
5.440 ( 1 H, d, $J_{H-3/H-5}$ = 1.3 Hz, H-3),
5.225 ( 1 H, dd, $J_{H\alpha}$/H-5 = 2 Hz, Hα),
5.008 ( 1 H, s, H-5),
3.923 ( 3 H, s, $OCH_3$).
75.46 MHz-$^{13}$C-NMR (breitbandentkoppelt):
C-2 (172.530), C-3 (90.268), C-4 (180.229) C-5 (79.872), C-α (66.721), $C_{Ar}$-1′ (138.409), $C_{Ar}$-2′ bis 6′(130.650, 129.571, 129.421, 129.032 und 127.265), $OCH_3$ (60.071).

Die Zuordnung der $^{13}C$-Signale wurde durch Offresonanz- und Gated-Spektrum sowie durch SFORD-Experimente gesichert.

Verfahrensvariante b:

Zur Lösung von 26.7 g (100 mmol) Ethyl-5-(2-Chlorphenyl)-3-methoxy-2(E),4(E)-pentadienoat in 450 ml Aceton gibt man 6.5 g (25 mmol) Tetraäthylammoniumacetat-Tetrahydrat und tropft unter Rühren und Kühlen auf 0°C nacheinander 25 ml 0.02M-Osmiumtetroxid-Lösung in tert. Butanol und 23.6 ml (170 mmol) 70%iges wäßriges tert. Butylhydroperoxid zu. Man läßt 12 Tage bei 4°C stehen, gibt 200 ml Dichlormethan und 140 ml 10%ige wäßrige Natriumsulfitlösung zu, rührt, bis kein Hydroperoxid mehr nachweisbar ist, trennt die organische Phase ab, extrahiert die wäßrige Phase mit 2 x 100 ml Dichlormethan und wäscht die vereinigten organ. Phasen mit wäßriger Kochsalzlösung. Die organische Phase enthält ein Gemisch von threo-Ethyl-5-(2-chlorphenyl)-4,5-dihydroxy-3-methoxy-2(E)-pentenoat und threo-5-(2-Chlorphenylhydroxymethyl)-4-methoxy-2(5H)-furanon. Durch Evaporieren vervollständigt man den Ringschluß und erhält nach Umkristallisation austert. Butylmethyläther 19.4 g (76.2 mmol) Reinprodukt mit Schmp. 149-151°C, das mit dem nach Beispiel Nr. 63 a gewonnenen Produkt dünnschichtchromatographisch und IR-spektroskopisch identisch ist. Ausbeute: 72.6%

Verfahrensvariante c:

Wie bei Variante b, jedoch wird statt des Ethylesters das Benzyl-5-(2-chlorphenyl)-3-methoxy-2(E),4(E)-pentadienoat eingesetzt. Ausbeute an threo-5-(2-Chlorphenylhydroxymethyl)-4-methoxy-2(5H)-furanon nach Umkristallisation aus Ethanol: 67.5%. Schmp. 149 - 151°C.

Verfahensvariante d:

Zur Lösung von 26.7 g (100 mmol) Ethyl-5-(2-Chlorphenyl)-3-methoxy-2(E),4(E)-pentadienoat in 360 ml Aceton gibt man unter Rühren 10 ml 0.02molare Osmiumtetroxid-Lösung in tert. Butanol und die Lösung von 14.9 g (110 mmol) N-Methylmorpholin-N-oxid-Hydrat in 30 ml Wasser und rührt 4 Tage bei Raumtemperatur. Durch Verrühren mit 5.2 g (50 mmol) Natriumbisulfit gelöst in 50 ml Wasser reduziert man überschüssiges N-Oxid, stellt mit 1M-Schwefelsäure auf pH=4 ein, zieht im Vakuum das Aceton ab und extrahiert die verbleibende wäßrige Mischung mit 2 x 200 ml dichlormethan. Der Extrakt wird mit verdünnter Schwefelsäure und Wasser frei von N-Methylmorpholin gewaschen und nach Trocknen über wasserfreiem Natriumsulfat im Vakuum konzentriert. Es kristallisieren 18.13 g threo-5-(2-Chlorphenylhydroxymethyl)-4-mthoxy-2(5H)-furanon aus. Evaporieren des Filtrats und Umkristallisation aus Ethylacetat ergeben weitere 1.20 g Reinprodukt. Ausbeute: 19.33 g (75.9 mmol) ≙ 75.9%.

Verfahrensvariante e:

Wie bei Variante d, jedoch wird die Reaktion statt in Aceton im 2-Phasensystem Butanon/Wasser durchgeführt und statt 110 mmol 160 mmol N-Methylmorpholin-N-oxid eingesetzt. Ausbeute: 76.2%

Verfahrensvariante f:

Wie bei Variante d, jedoch im 2-Phasensystem Dichlormethan / Wasser. Ausbeute: 66.7%

Verfahrensvariante g:

Das Gemisch aus 23.6 g (80 mmol) sec. Butyl-5-(2-chlorphenyl)-3-methoxy-2(E),4(E)-pentadienoat, 200 ml Butanon, 15 ml 0.02M-Osmiumtetroxid-Lösung in tert. Butanol, 17.6 g (160 mmol) N-Methylmorpholin-N-oxid-Hydrat und 50 ml Wasser wird 7 Tage bei Raumtemperatur gerührt. Nach Zugabe von 100 ml Dichlormethan, verrührt man mit 100 ml 2%iger wäßriger Natriumbisulfit-Lösung, trennt die organische Phase ab und wäscht sie mit 2 mal 100 ml 0.5M-Salzsäure und 5 mal mit 100 ml Wasser. Die organische Phase enthält ein Gemisch von threo-sec.Butyl-5-(2-chlorphenyl)-4.5-dihydroxy-3-methoxy-2(E)-pentenoat und threo-5-(2-Chlorphenylhydroxymethyl)-4-methoxy-2(5H)-furanon. Durch Zugabe von 0.1 ml 32% Salzsäure und Evaporieren der organischen Phase vervollständigt man den Ringschluß und erhält nach Umkristallisieren aus Ether 10.66 g (41.9 mmol) threo-5-(2-Chlorphenylhydroxymethyl)-4-methoxy-2(5H)-furanon. Ausbeute: 52.3%.

In gleicher Weise kann man statt des sec. Butylesters die entsprechenden Methyl-, Isopropyl- oder tert.Butylester einsetzen.

Analog zu den in Beispiel 64 beschriebenen Verfahrensvarianten a bis g werden durch Oxidation der in den Beispielen Nr. 6 bis Nr. 63 beschriebenen 3-Alkoxy-5-phenyl-2(E),4(E)-pentadiensäure-Derivate folgende threo-4-Alkoxy-5-phenylhydroxymethyl-2(5H)-furanon-Derivate hergestellt: siehe Tabelle 6.

Tabelle 7 enthält die Ergebnisse der Elementaranalysen, Tabelle 8 die 1H-NMR-spektroskopischen Daten.

Tabelle 6: threo-4-Alkoxy-5-phenylhydroxymethyl-2(5H)-furanone

| Beispiel Nr. | Bezeichnung | Variante | Ausb. [%] | Schmp. [°C] (umkristallisiert aus) |
|---|---|---|---|---|
| 65 | threo-4-Methoxy-5-phenylhydroxymethyl-2(5H)-furanon | a<br>b<br>d | 46<br>49<br>59 | 157 (Dichlormethan) |
| 66 | threo-5-(2-Chlorphenylhydroxymethyl)-4-ethoxy-2(5H)-furanon | a | 24 | 114-116 (Isopropanol) |
| 67 | threo-5-(4-Chlorphenylhydroxymethyl)-4-methoxy-2(5H)-furanon | b | 47 | 148-150 (Dichlormethan) |
| 68 | threo-5-(2-Bromphenylhydroxymethyl)-4-methoxy-2(5H)-furanon | d | 85 | 162-164 (Dichlormethan) |
| 69 | threo-5-(3-Bromphenylhydroxymethyl)-4-methoxy-2(5H)-furanon | b | 50 | 167-168 (Aceton / Ethanol) |
| 70 | threo-5-(2-Fluorphenylhydroxymethyl)-4-methoxy-2(5H)-furanon | d | 87 | 130-133 (Dichlormethan) |
| 71 | threo-4-Methoxy-5-(2-trifluormethylphenylhydroxymethyl)-2(5H)-furanon | b | 62 | 166-167 (Ethanol / Wasser) |
| 72 | threo-4-Methoxy-5-(3-trifluormethylphenylhydroxymethyl)-2(5H)-furanon | b | 27 | 128-129 (Ethanol / Wasser) |
| 73 | threo-4-Methoxy-5-(4-trifluormethylphenylhydroxymethyl)-2(5H)-furanon | b | 28 | 171 (Methanol) |
| 74 | threo-4-Methoxy-5-(2-nitrophenylhydroxymethyl)-2(5H)-furanon | a | 10 | 170-172 (Ethylacetat) |

1. Fortsetzung Tabelle 6

| Beispiel Nr. | Bezeichnung | Variante | Ausb. [%] | Schmp. [°C] (umkristallisiert aus) |
|---|---|---|---|---|
| 75 | threo-4-Methoxy-5-(3-nitrophenylhydroxymethyl)-2(5H)-furanon | a | 21 | 149-151 (Ethylacetat) |
| 76 | threo-5-(2.5-Dimethylphenylhydroxymethyl)-4-methoxy-2(5H)-furanon | b | 47 | 171-173 (Ethanol) |
| 77 | threo-5-[(2-Chlor-5-methylphenyl)-hydroxymethyl]-4-methoxy-2(5H)-furanon | b | 71 | 174-175 (Methanol) |
| 78 | threo-5-(2.3-Dichlorphenylhydroxymethyl)-4-methoxy-2(5H)-furanon | e | 75 | 161-162 (Methanol) |
| 79 | threo-5-(2.4-Dichlorphenylhydroxymethyl)-4-methoxy-2(5H)-furanon | a<br>b<br>e | 38<br>87<br>55 | 174 (Dichlormethan) |
| 80 | threo-5-(2.4-Dichlorphenylhydroxymethyl)-4-ethoxy-2(5H)-furanon | b | 72 | 133 (Isopropanol) |
| 81 | threo-5-(2.5-Dichlorphenylhydroxymethyl)-4-methoxy-2(5H)-furanon | a<br>b | 15<br>70 | 187 (Dichlormethan) |
| 82 | threo-5-(2.5-Dichlorphenylhydroxymethyl)-4-ethoxy-2(5H)-furanon | b | 70 | 175-178 (Isopropanol) |
| 83 | threo-5-(3.4-Dichlorphenylhydroxymethyl)-4-methoxy-2(5H)-furanon | b | 53 | 158-159 (Ethylacetat) |
| 84 | threo-5-(3.5-Dichlorphenylhydroxymethyl)-4-methoxy-2(5H)-furanon | b | 62 | 177-179 (Ethanol) |

2. Fortsetzung von Tabelle 6

| Beispiel Nr. | Bezeichnung | Variante | Ausb. [%] | Schmp. [°C] (umkristallisiert aus) |
|---|---|---|---|---|
| 85 | threo-5-[3.5-Bis-(trifluormethyl)-phenylhydroxymethyl]-4-methoxy-2(5H)-furanon | e | 52 | 191-194 (Ethylacetat) |
| 86 | threo-5-[(2-Chlor-5-trifluormethylphenyl)-hydroxymethyl]-4-methoxy-2(5H)-furanon | e | 69 | 196-199 (Ethylacetat) |
| 87 | threo-5-[(4-Chlor-2-trifluormethylphenyl)-hydroxymethyl]-4-methoxy-2(5H)-furanon | e | 76 | 166-172 (Ethylacetat) |
| 88 | threo-5-[(4-Brom-2-chlorphenyl)-hydroxymethyl]-4-methoxy-2(5H)-furanon | e | 84 | 173-176 (Methanol) |

Tabelle 7: Elementaranalysen

| Beispiel Nr. | Summenformel / Molmasse | % Ber. C / % Gef. C | H / H | Sonstige / Sonstige |
|---|---|---|---|---|
| 65 | $C_{12}H_{12}O_4$ | 65.45 | 5.49 | |
| | 220.23 | 65.59 | 5.59 | |
| 66 | $C_{13}H_{13}ClO_4$ | 58.11 | 4.88 | Cl (13.19) |
| | 268.69 | 58.03 | 4.93 | 13.5 |
| 67 | $C_{12}H_{11}ClO_4$ | 56.59 | 4.35 | Cl (13.92) |
| | 254.67 | 56.26 | 4.14 | 14.1 |
| 68 | $C_{12}H_{11}BrO_4$ | 48.18 | 3.71 | Br (26.71) |
| | 299.13 | 48.18 | 3.65 | 27.2 |
| 69 | $C_{12}H_{11}BrO_4$ | 48.18 | 3.71 | Br (26.71) |
| | 299.13 | 48.24 | 3.81 | 27.5 |
| 70 | $C_{12}H_{11}FO_4$ | 60.50 | 4.66 | F (7.98) |
| | 238.22 | 60.22 | 4.29 | 7.6 |
| 71 | $C_{13}H_{11}F_3O_4$ | 54.17 | 3.85 | F (19.78) |
| | 288.23 | 54.37 | 4.02 | |
| 72 | $C_{13}H_{11}F_3O_4$ | 54.17 | 3.85 | F (19.78) |
| | 288.23 | 54.25 | 4.09 | 20.0 |
| 73 | $C_{13}H_{11}F_3O_4$ | 54.17 | 3.85 | F (19.78) |
| | 288.23 | 54.92 | 4.02 | 19.0 |
| 74 | $C_{12}H_{11}NO_6$ | 54.34 | 4.18 | N (5.28) |
| | 265.23 | 54.48 | 4.29 | 5.15 |
| 75 | $C_{12}H_{11}NO_6$ | 54.34 | 4.18 | N (5.28) |
| | 265.23 | 54.26 | 4.28 | 5.45 |
| 76 | $C_{14}H_{16}O_4$ | 67.73 | 6.50 | |
| | 248.29 | 67.78 | 6.88 | |
| 77 | $C_{13}H_{13}ClO_4$ | 58.11 | 4.88 | Cl (13.19) |
| | 268.69 | 58.00 | 4.90 | 13.8 |
| 78 | $C_{12}H_{10}Cl_2O_4$ | 49.85 | 3.49 | Cl (24.53) |
| | 289.12 | 49.86 | 3.46 | 24.8 |
| 79 | $C_{12}H_{10}Cl_2O_4$ | 49.85 | 3.49 | Cl (24.53) |
| | 289.12 | 49.89 | 3.46 | 24.6 |
| 80 | $C_{13}H_{12}Cl_2O_4$ | 51.51 | 3.99 | Cl (23.39) |
| | 303.15 | 51.23 | 3.94 | 23.0 |

Fortzetzung von Tabelle 7

| Beispiel Nr. | Summenformel / Molmasse | % Ber. C / % Gef. C | H / H | Sonstige / Sonstige |
|---|---|---|---|---|
| 81 | $C_{12}H_{10}Cl_2O_4$ | 49.85 | 3.49 | Cl (24.53) |
| | 289.12 | 49.62 | 3.35 | 24.6 |
| 82 | $C_{13}H_{12}Cl_2O_4$ | 51.51 | 3.99 | Cl (23.39) |
| | 303.15 | 51.09 | 3.84 | 23.0 |
| 83 | $C_{12}H_{10}Cl_2O_4$ | 49.85 | 3.49 | Cl (24.53) |
| | 289.12 | 49.62 | 3.36 | 24.3 |
| 84 | $C_{12}H_{10}Cl_2O_4$ | 49.85 | 3.49 | Cl (24.53) |
| | 289.12 | 49.82 | 3.70 | 24.6 |
| 85 | $C_{14}H_{10}F_6O_4$ | 47.21 | 2.83 | F (32.00) |
| | 356.23 | 47.16 | 2.90 | |
| 86 | $C_{13}H_{10}ClF_3O_4$ | 48.39 | 3.12 | Cl (10.99) |
| | 322.67 | 47.88 | 3.00 | 10.8 |
| 87 | $C_{13}H_{10}ClF_3O_4$ | 48.39 | 3.12 | Cl (10.99) |
| | 322.67 | 48.34 | 3.05 | 11.3 |
| 88 | $C_{12}H_{10}BrClO_4$ | 43.21 | 3.02 | Br (23.96) |
| | 333.57 | 43.12 | 2.95 | 23.5 |
| | | | | Cl (10.63) |
| | | | | 10.2 |

Tabelle 8: 300 MHz -$^1$H-NMR, $\delta$ [ppm]

| Beispiel Nr. | 4-$OCH_3$ (s) | H-3 | H-5 | H-α | α-OH |
|---|---|---|---|---|---|
| | | $J_{3/5}$ [Hz] | $J_{5/\alpha}$ [Hz] | $J_{\alpha/OH}$ [Hz] | |
| 65 | 3.88 | 5.369 (s) | 5.091(d) | 4.903 (t) | 5.740 (d) |
| | | - | 1.7 | 5.75 | |
| 66 | 4.149(m) (4-O-$CH_2$-) | 5.364 (d) | 4.960(dd) | 5.218(dd) | 5.866 (d) |
| | | 1.17 | 2.35 | 5.28 | |
| 67 | 3.88 | 5.378 (s) | 5.095(d) | 4.92 (t) | 5.864 (d) |
| | | - | ca.1.5 | 5.3 | |
| 68 | 3.992 | 5.437 (s) | 5.000(d) | 5.181(t) | 5.972 (d) |
| | | - | 1.47 | 5.28 | |
| 69 | 3.898 | 5.393 (d) | 5.137(d) | 4.928(dd) | 5.877 (d) |
| | | 0.89 | 1.33 | 5.75 | |
| 70 | 3.904 | 5.412 (d) | 4.993(d) | 5.159(dd) | 5.877 (d) |
| | | 0.89 | 1.3 | 5.75 | |
| 71 | 3.898 | 5.452 (s) | 4.861(s) | 5.167(bs) | 6.099 (d) |
| | | - | - | 5.31 | |
| 72 | 3.89 | 5.344 (d) | 5.150(dd) | 5.028(dd) | 5.869 (d) |
| | | 0.89 | 2.21 | 5.31 | |
| 73 | 3.90 | 5.353 (d) | 5.130(dd) | 5.015(dd) | 5.849 (d) |
| | | 0.89 | 2.21 | 5.75 | |
| 75 | 3.917 | 5.419(d) | 5.230(dd) | 5.108(dd) | 6.107 (d) |
| | | 0.88 | 2.21 | 5.75 | |
| 76 | 3.90 | 5.380 (d) | 4.987(bs) | 5.031(dd) | 5.551 (d) |
| | | 0.89 | 1.77 | 4.87 | |
| 77 | 3.910 | 5.387 (d) | 4.966(dd) | 5.191(dd) | 5.798 (d) |
| | | 0.88 | 1.77 | 5.75 | |
| 78 | 3.92 | 5.407 (d) | 5.022 (dd) | 5.259 (dd) | 5.993 (d) |
| | | 1.17 | 2.34 | 5.29 | |
| 79 | 3.92 | 5.446 (s) | 5.005(bs) | 5.186(dd) | 6.064 (d) |
| | | - | 1.77 | 5.75 | |
| 80 | 4.15 (m) (4-O-$CH_2$-) | 5.336 (d) | 4.954(dd) | 5.179(dd) | 5.958 (d) |
| | | 0.89 | 2.65 | 5.75 | |
| 81 | 3.925 | 5.458 (s) | 5.035(bs) | 5.175(dd) | 6.131 (d) |
| | | - | 1.77 | 5.75 | |
| 82 | 4.160 (m) (4-O-$CH_2$-) | 5.380 (d) | 4.989(dd) | 5.174(dd) | 6.035 (d) |
| | | 0.89 | 2.65 | 5.75 | |

Fortsetzung Tabelle 8

| Beispiel Nr. | 4-$OCH_3$ (s) | H-3 $J_{3/5}$[Hz] | H-5 $J_{5/\alpha}$[Hz] | H-α $J_{\alpha/OH}$[Hz] | α-OH |
|---|---|---|---|---|---|
| 83 | 3.901 | 5.400(s) - | 5.153(d) 1.77 | 4.96 (dd) 5.75 | 5.976 (d) |
| 84 | 3.897 | 5.363(d) 0.89 | 5.152(t) 2.21 | 4.952(t) 5.75 | 5.941 (d) |
| 86 | 3.93 | 5.428(d) 1.33 | 5.060(t) 1.77 | 5.276(dd) 5.75 | 6.134 (d) |
| 88 | 3.91 | 5.399(d) 1.17 | 4.977(d) 2.34 | 5.179(dd) 5.87 | 5.951 (d) |

Abkürzungen: s = singulett, bs = verbreitertes singulett,
d = dublett, dd=doppeltes dublett, t=triplett
m = multiplett

Beispiel Nr. 89

threo-5-(2-Chlorphenylhydroxymethyl)-4-methoxy-5-methyl-2(5H)-furanon:

Verfahren A:

Das Gemisch aus 28 g Ethyl-5-(2-Chlorphenyl)-3-methoxy-4-methyl-2(E),4(E)-pentadienoat (Rohprodukt aus Beispiel Nr. 61), 250 ml Tetrahydrofuran und 50 ml Wasser wird mit 25 ml 0.02M-Osmiumtetroxid-Lösung in tert. Butanol und 10.7 g Natriumchlorat versetzt und 4 Tage unter Rühren zum Rückfluß erhitzt. Man gibt erneut 25 ml 0.02M-Osmiumtetroxid-Lösung und 10.7 g Natriumchlorat zu und kocht weitere 3 Tage unter Rückfluß. Trotz unvollständiger Oxidation arbeitet man auf. Nach Abkühlen und Zugabe von 100 ml Dichlormethan wäscht man anorganische Bestandteile mit Wasser heraus, trocknet die Dichlormethanphase über Natriumsulfat, evaporiert und kristallisiert aus Ethylacetat um. Man erhält 1.463 g reines threo-5-(2-Chlorphenylhydroxymethyl)-4-methoxy-5-methyl-2(5H)-furanon mit Schmp. 181 - 184°C. Das DC an Kieselgel F 254 mit Chloroform/Methanol 95 / 5 ergibt Rf = 0.37.
Analyse: $C_{13}H_{13}ClO_4$: (268.69)
Ber.: C (58.11), H (4.88), Cl (13.19)
Gef.: C (57.52), H (4.67), Cl (12.9)

Verfahren B:

Unter Rühren, Kühlen auf -77°C und Stickstoffatmosphäre tropft man zu der Lösung aus 8.30 g (82 mmol) Diisopropylamin in 150 ml Tetrahydrofuran 50 ml einer 1.64-molaren Lösung von n-Butyllithium in Hexan und 10.52 g (82 mmol) 1.3-Dimethyl-3.4.5.6-tetrahydro-2(1H)-pyrimidinon (DMPU). Anschließend tropft man die Lösung von 10.5 g (82 mmol) 4-Methoxy-5-methyl-2(5H)-furanon in 80 ml Tetrahydrofuran zu, rührt 30 Min. und tropft die Lösung von 11.84 g (82 mmol) 2-Chlorbenzaldehyd in 50 ml Tetrahydrofuran zu. Unter Rühren läßt man innerhalb 16 Stdn. auf 0°C kommen, hydrolysiert durch Zutropfen von 50 ml Wasser und stellt mit 2M-Salzsäure auf pH = 5.5 ein. Die organische Phase wird abgetrennt und evaporiert, die wäßrige Phase 3 mal mit 100 ml Ether extrahiert. Evaporat und Etherphasen werden vereinigt, mit Wasser frei von DMPU gewaschen, über Natriumsulfat getrocknet und evaporiert. Der Rückstand wird an 400 g Kieselgel mit Chloroform / Methanol 98 / 2 als Eluens säulenchromatographisch aufgetrennt. Die Fraktionen mit Rf (Chloroform / Methanol 95 / 5) = 0.37 ergeben nach Evaporieren und Umkristallisation aus tert. Butylmethylether / Dichlormethan 5.3 g (19.7 mmol) reines threo-5-(2-Chlorphenylhydroxymethyl)-4-methoxy-5-methyl-2(5H)-furanon mit Schmp. 192 - 194°C, das dünnschichtchromatographisch und IR-spektroskopisch identisch mit dem nach Verfahren A erhaltenen Produkt ist.
analyse: $C_{13}H_{13}ClO_4$ (268.69)
Ber.: C (58.11), H (4.88), Cl (13.19)
Gef.: C (58.05), H (4.81), Cl (13.2)

Die Fraktionen mit Rf = 0.48 ergeben nach Evaporieren und Umkristallisation aus Toluol 2.64 g (9.84 mmol) reines erythro-5-(2-Chlorphenylhydroxymethyl)-4-methoxy-5-methyl-2(5H)-furanon mit Schmp. 153-155°C.
Analyse: $C_{13}H_{13}ClO_4$ (268.69)
Ber.: C (58.11), H (4.88), Cl (13.19)
C (58.02), H (4.87), Cl (13.3)

Beispiel Nr. 90

threo-5-(2-Chlorphenylhydroxymethyl)-4-methoxy 2(5H)-furanon:

Unter Kühlen auf –70°C, Rühren und Stickstoffatmosphäre tropft man zu der Lösung von 49.9 ml (327 mmol) Diisopropylamin in 500 ml wasserfreiem Tetrahydrofuran 200 ml 1.64-molare Lösung von n-Butyllithium in Hexan, 84 g (655 mmol) 1.3-Dimethyl-3.4.5.6-tetrahydro-2(1H)-pyrimidinon und die Lösung von 37.4 g (327 mmol) 4-Methoxy-2(5H)-furanon in 300 ml Tetrahydrofuran. Man rührt 30 Min. bei –55°C nach, tropft die Lösung von 46 g (327 mmol) 2-Chlorbenzaldehyd und 5.8 ml Wasser in 100 ml Tetrahydrofuran zu und rührt 16 Stdn. unter allmählicher Erwärmung auf 0°C nach. Nach Hydrolyse mit 200 ml Wasser, Einstellung auf pH = 5.5 mit 2M-Salzsäure und Phasentrennung evaporiert man die organische Phase, extrahiert die wäßrige Phase mit 2 mal 300 ml Ether, vereinigt Evaporat und Etherphasen, wäscht mit 3 mal 300 ml Wasser und evaporiert. Das ölige Rohprodukt, bestehend aus dem Gemisch von threo- und erythro-Isomer, wird nach Verreiben mit Tetrachlormethan kristallin. Mehrfach fraktionierte Umkristallisation aus Ethylacetat ergibt 41.5 g (163 mmol) reines threo-5-(2-Chlorphenylhydroxymethyl)-4-methoxy-2(5H)-furanon mit Schmp. 140 - 145°C und Rf = 0.35 (DC, Kieselgel F 254; Chlorform/Methanol 95/5). Das Produkt ist dünnschichtchromatographisch, NMR- und IR-spektroskopisch identisch mit dem in Beispiel Nr. 64 hergestellten.
Aus den Mutterlaugen der Kristallisation erhält man 5.0 g (20 mmol) reines erythro-5-(2-Chlorphenylhy-

droxymethyl)-4-methoxy-2(5H)-furanon mit Schmp. 159 - 161°C (Ethylacetat) und Rf = 0.45.
Analyse: $C_{12}H_{11}ClO_4$ (254.67)
Ber.: C (56.59), H (4.35), Cl (13.92)
Gef.: C (56.80), H (4.50), Cl (13.9 )
300 MHz-$^1$H-NMR: 7.27 - 7.67 (4 H, m, aromat.Protonen), 6.205 (1 H, d, $J_{OH/H\alpha}$ = 5.0 Hz, α-OH) 5.310 (1 H, s, H-3), 5.292 (1 H, dd, $J_{H\alpha/H-5}$ = 2.7 Hz, Hα) 5.113 (1 H, d, H-5), 3.73 (3 H, s, $OCH_3$)

Weitere Konzentration der Mutterlaugen ergibt noch 18.7 g (73 mmol) kristallines Gemisch aus threo- und erythro-Isomer, das nicht mehr aufgetrennt wurde.

Beispiel Nr. 91

4-Ethoxy-2(5H)-furanon:

Das Gemisch aus 34.2 g (300 mmol) 4-Methoxy-2(5H)-furanon, 200 ml Ethanol und 30 mmol Natriumethoxid wird im Autoklaven unter Stickstoffatmosphäre 24 Stdn. bei 120°C gerührt. Nach dem Abkühlen filtriert man über 100 g Kieselgel, evaporiert das Filtrat und destilliert 2 mal bei 10 mbar über eine Vigreux-Kolonne. Man erhält 21.6 g (169 mmol ) 4-Ethoxy-2(5H)-furanon mit $Kp_{10}$ = 135-137°C.
Ausbeute: 56%

Beispiel Nr. 92

4-Propoxy-2(5H)-furanon:

Herstellung analog Beispiel Nr. 91 aus 4-Methoxy-2(5H)-furanon und Propanol. $Kp_{10}$ = 143 - 145°C.
Ausbeute: 39 %
Analyse: $C_7H_{10}O_3$ (142.16)
Ber.: C (59.14), H (7.09)
Gef.: C (59.18), H (7.36)

Beispiele Nr. 93 - 105

Analog zu den in Beispielen Nr.89 B u.90 angegebenen Verfahren wurden weitere threo-4-Alkoxy-5-[α-hydroxy-ω-(subst.)phenylalkyl]-2(5H)-furanone hergestellt und von den sie begleitenden erythro-Isomeren getrennt. Bezeichnung, Schmelzpunkte und dünnschichtchromatographisch an Kieselgel F 254 ermittelte Rf-Werte sind in Tabelle 9, analytische Daten in Tabelle 10 und $^1$H-NMR-spektroskopische Daten in Tabelle 11 zusammengefaßt. Die Daten der erythro-Isomeren sind, soweit vorhanden, mit aufgeführt.

Tabelle 9: threo-4-alkoxy-5-[α-hydroxy-ω-(subst.)-phenylalkyl]-2(5H)-furanone

| Beisp. Nr. | Bezeichnung | Rf (Eluens) | Schmp.[°C] (umkrist.aus) |
|---|---|---|---|
| 93 | threo-4-Methoxy-5-(2-methylphenylhydroxymethyl)-2(5H)-furanon | 0.45 (Aceton/Toluol 1/1) | 174-176 (Isopropanol) |
| 94 | threo-4-Methoxy-5-(3-methylphenylhydroxymethyl)-2(5H)-furanon | 0.39 (Toluol/Isopropanol 5 / 1) | 127-130 (Dichlormethan/Pentan) |
| | [erythro-Isomeres: | 0.45 | 139-141] (Toluol) |
| 95 | threo-4-Methoxy-5-(4-methylphenylhydroxymethyl)-2(5H)-furanon | 0.45 (Toluol/Aceton 1/1) | 148-151.5 (Isopropanol/Pentan) |
| | [erythro-Isomeres: | 0.47 | 143-147] (Isopropanol) |
| 96 | threo-5-(3-Fluorphenylhydroxymethyl)-4-methoxy-2(5H)-furanon | 0.30 ($CHCl_3$/$CH_3OH$ 95/5) | 123-126 (Dichlormethan/Pentan) |
| 97 | threo-5-(4-Fluorphenylhydroxymethyl)-4-methoxy-2(5H)-furanon | 0.26 ($CHCl_3$/$CH_3OH$ 98/2) | 141 (Toluol/tert.Butylmethylether) |
| | [erythro-Isomeres: | 0.26 | 141-143 ] (Toluol/tert.Butylmethylether) |
| 98 | threo-5-(3-Chlorphenylhydroxymethyl)-4-methoxy-2(5H)-furanon | 0.41 (Toluol/Aceton 1/1) | 150-152 (Chloroform / Pentan) |
| | [erythro-Isomeres | 0.45 | 121-126] (Chloroform / Pentan) |

Fortsetzung Tabelle 9

| Beisp. Nr. | Bezeichnung | Rf (Eluens) | Schmp.[°C] (umkrist.aus) |
|---|---|---|---|
| 99 | threo-5-(2-Chlorphenylhydroxymethyl)-4-propoxy-2(5H)-furanon | 0.39 ($CHCl_3$/$CH_3OH$ 98/2) | 103-105 ($CCl_4$) |
| | [erythro-Isomeres : | 0.39 | 147-149 ] (Ethylacetat) |
| 100 | threo-5-(4-Bromphenylhydroxymethyl)-4-methoxy-2(5H)-furanon | 0.46 (Toluol/Aceton 1/1) | 157-158 ($CCl_4$/Ethylacetat) |
| | [erythro-Isomeres: | 0.49 | 149-150 ] (Ethylacetat) |
| 101 | threo-4-Methoxy-5-(4-nitrophenylhydroxymethyl)-2(5H)-furanon | 0.42 (Toluol/Aceton 1/1) | 226-228 (Isopropanol) |
| | [erythro-Isomeres: | 0.45 | 180-184] (Ethylacetat) |
| 102 | threo-5-(2-Chlor-6-fluorphenylhydroxymethyl)4-methoxy-2(5H)-furanon | 0.58 ($CHCl_3$/$CH_3OH$ 95/5) | 129-133 ($CCl_4$/Pentan) |
| | [erythro-Isomeres: | 0.54 | 151-155 ] ($CCl_4$-Isopropanol) |
| 103 | threo-5-(2.6-Dichlorphenylhydroxymethyl)-4-methoxy-2(5H)-furanon | 0.54 ($CHCl_3$/$CH_3OH$ 95/5) | 176-180 (Toluol/Aceton) |
| | [erythro-Isomeres: | 0.63 | 153-158 ] (Ethylacetat) |
| 104 | threo-4-Methoxy-5-(α-hydroxy-β-phenylethyl)-2(5H)-furanon (verunreinigt durch erythro-Isomeres) | 0.8 ($CHCl_3$/$CH_3OH$ 7/3) | 79-84 (Benzol) |
| 105 | threo-4-Methoxy-5-(α-hydroxy-γ-phenylpropyl)-2(5H)-furanon | 0.45 (Aceton/Toluol 1/1) | 179-181 ($CH_3OH$/Aceton) |

Tabelle 10: Elementaranalysen

| Beispiel Nr. | Summenformel / Molmasse | % Ber. C / % Gef. C | H / H | Sonstige / Sonstige |
|---|---|---|---|---|
| 93 | $C_{13}H_{14}O_4$ | 66.66 | 6.02 | |
| | 234.25 | 66.91 | 6.42 | |
| 94 | $C_{13}H_{14}O_4$ | 66.66 | 6.02 | |
| | 234.25 | 66.62 | 6.43 | |
| | (erythro | 66.87 | 6.03 ) | |
| 95 | $C_{13}H_{14}O_4$ | 66.66 | 6.02 | |
| | 234.25 | 66.54 | 6.38 | |
| | (erythro: | 66.92 | 6.10 ) | |
| 96 | $C_{12}H_{11}FO_4$ | 60.50 | 4.66 | F (7.98) |
| | 238.22 | 60.55 | 4.90 | 7.50 |
| 97 | $C_{12}H_{11}FO_4$ | 60.50 | 4.66 | F (7.98) |
| | 238.22 | 60.31 | 4.59 | 7.6 |
| | (erythro: | 60.39 | 4.75 | 7.6 ) |
| 98 | $C_{12}H_{11}ClO_4$ | 56.59 | 4.35 | Cl (13.92) |
| | 254.67 | 56.11 | 4.33 | 14.6 |
| | (erythro: | 56.50 | 4.32 | 14.3 ) |
| 99 | $C_{14}H_{15}ClO_4$ | 59.48 | 5.35 | Cl (12.54) |
| | 282.73 | 59.36 | 5.44 | |
| | (erythro: | 59.80 | 5.52 | 12.5 ) |
| 100 | $C_{12}H_{11}BrO_4$ | 48.18 | 3.71 | Br (26.71) |
| | 299.13 | 47.92 | 3.80 | 26.8 |
| | (erythro: | 47.28 | 3.67 | 26.6) |
| 101 | $C_{12}H_{11}NO_6$ | 54.34 | 4.18 | N (5.28) |
| | 265.23 | 54.77 | 4.15 | 5.13 |
| | (erythro: | 54.27 | 4.24 | 5.08 ) |
| 102 | $C_{12}H_{10}ClFO_4$ | 52.86 | 3.70 | Cl (13.00) |
| | 272.66 | 52.71 | 3.60 | 13.1 |
| | (erythro: | 52.02 | 3.63 | 13.5) |
| 103 | $C_{12}H_{10}Cl_2O_4$ | 49.85 | 3.49 | Cl (24.53) |
| | 289.12 | 49.78 | 3.45 | 24.4 |
| | (erythro: | 49.77 | 3.57 | 24.4 ) |

Fortsetzung Tabelle 10

| Beispiel Nr. | Summenformel / Molmasse | % Ber. C / % Gef. C | H / H | Sonstige / Sonstige |
|---|---|---|---|---|
| 104 | $C_{13}H_{14}O$ | 66.66 | 6.02 | |
| | 234.25 | 66.06 | 6.16 | |
| 105 | $C_{14}H_{16}O_4$ | 67.73 | 6.50 | |
| | 248.29 | 68.11 | 6.70 | |

Tabelle 11: 300 MHz-$^1$H-NMR, $\delta$ [ppm]

| Beispiel Nr. | 4-$OCH_3$ (s) | H-3<br>$J_{3/5}$[Hz] | H-5<br>$J_{5/\alpha}$[Hz] | Hα<br>$J_{\alpha/OH}$[Hz] | α-OH |
|---|---|---|---|---|---|
| 93 | 3.904 | 5.390 (d)<br>0.88 | 5.015(bs)<br>1.77 | 5.07(dd)<br>5.31 | 5.599(d) |
| 94 | 3.89 | 5.36 (d)<br>0.8 | 5.07 (d)<br>2 | 4.86(dd)<br>6 | 5.68 (d) |
| [erythro: | 3.75 | 5.15 (s)<br>- | 5.14 (d)<br>3.0 | 4.89(dd)<br>4.9 | 5.88 (d)] |
| 95 | 3.89 | 5.35 (d)<br>0.8 | 5.04 (d)<br>2.2 | 4.86(dd)<br>5.6 | 5.65 (d) |
| [erythro: | 3.75 | 5.144 (s)<br>- | 5.138 (d)<br>3 | 4.89(dd)<br>4.9 | 5.87 (d)] |
| 96 | 3.90 | 5.39 (d)<br>0.9 | 5.14 (dd)<br>2 | 4.94(dd)<br>5.6 | 5.87 (d) |
| 97 | 3.90 | 5.36 (d)<br>0.89 | 5.07 (dd)<br>2.21 | 4.91(dd)<br>5.31 | 5.78 (d) |
| [erythro: | 3.77 | 5.18 (d)<br>0.89 | 5.16 (d)<br>3.10 | 4.96(dd)<br>4.87 | 6.02 (d)] |
| 98 | 3.87 | 5.39 (d)<br>0.88 | 5.14 (dd)<br>2.2 | 4.94(dd)<br>5.75 | 5.86 (d) |
| [erythro: | 3.76 | 5.23 (d)<br>0.88 | 5.20 (d)<br>2.7 | 4.98(dd)<br>5.3 | 6.14 (d)] |
| 99 | 4.06 (m)<br>(4-O-$CH_2$-) | 5.42 (d)<br>1 | 4.99 (dd)<br>2.5 | 5.22(dd)<br>5.8 | 5.97 (d) |
| [erythro: | 3.88 (t) | 5.29 (s)<br>- | 5.10 (d)<br>2 | 5.30(dd)<br>5 | 6.21 (d)] |
| 100 | 3.89 | 5.37 (d)<br>0.88 | 5.09 (d)<br>1.7 | 4.90(dd)<br>5.31 | 5.83 (d) |
| [erythro: | 3.76 | 5.19 (s)<br>- | 5.17 (d)<br>2.21 | 4.94(dd)<br>5.15 | 6.05 (d)] |
| 101 | 3.91 | 5.41 (d)<br>0.89 | 5.20 (dd)<br>1.77 | 5.08(dd)<br>5.75 | 6.07 (d) |
| [erythro: | 3.77 | 5.25 (s)<br>- | 5.26 (d)<br>2.65 | 5.14(dd)<br>5.31 | 6.32 (d)] |

Fortzetzung von Tabelle 11

| Beispiel Nr | 4-$OCH_3$ (s) | H-3 | H-5 | Hα | α-OH |
|---|---|---|---|---|---|
| | | $J_{3/5}$[Hz] | $J_{5/\alpha}$[Hz] | $J_{\alpha/OH}$[Hz] | |
| 102 | 3.73 | 5.45 (s)<br>- | 5.12 (d)<br>6.19 | 5.16(t)<br>4.87 | 6.19 (d) |
| [erythro: | 3.86 | 5.38 (s) | 5.15 (d)<br>6.19 | 5.24(t)<br>5.75 | 6.21 (d)] |
| 103 | 3.99 | 5.41 (s) | 5.32 (d)<br>7.96 | 5.37(dd)<br>4.87 | 6.22 (d) |
| [erythro: | 3.64 | 5.44 (s) | 5.35 (d)<br>7.08 | 5.27(dd)<br>5.31 | 6.27 (d)] |
| 105 | 3.85 | 5.33 (d)<br>0.9 | 4.85 (t)<br>ca.1.8 | 3.67(m) | 5 (m) |

Abkürzungen wie bei Tabelle 8

Beispiel Nr. 106

threo-4-Ethoxy-5-[methoxy-(2.4-dichlorphenyl)-methyl]-2(5H)-furanon:

Das Gemisch aus 3.0 g (9.9 mmol) threo-5-(2.4-Dichlorphenylhydroxymethyl)-4-ethoxy-2(5H)-furanon, 50 ml Butanon, 9.23 g (39.8 mmol) Silber(I)oxid und 5 ml (80 mmol) Jodmethan wird 24 Stdn. unter Rühren zum Sieden unter Rückfluß erhitzt. Man filtriert, evaporiert das Filtrat und kristallisiert das ölige Rohprodukt 2 mal aus Ether / Petrolether um. Man erhält 2.03 g (6.4 mmol) threo-4-Ethoxy-5-[methoxy-(2.4-dichlorphenyl)-methyl]-2(5H)-furanon mit Schmp. 110 - 114°C.
Ausbeute: 64.6%.
Analyse: $C_{14}H_{14}Cl_2O_4$ (317.18)
Ber.: C (53.02), H (4.45), Cl (22.35)
Gef.: C (53.16), H (4.60), Cl (22.4 )
300 MHz-$^1$H-NMR: 7.45-7.7 (3 H, m, aromat.Protonen)
5.40 (1 H, d, $J_{H-3/H-5}$ = 0.88 Hz,H-3)
5.05 (1 H, dd, $J_{H-5/H\alpha}$ = 3.10 Hz; H-5),
4.86 (1 H, d, Hα),
4.17 (2 H, $ABX_3$-System, 4-$OCH_2$-),

3.20 ( 3 H, s, α-$OCH_3$),
1.30 (3 H, $J_{CH_3/OCH_2}$ = 7.07 Hz, $CH_3$).

Beispiel Nr. 107

threo-4-Methoxy-5-[methoxymethoxy-(2-chlorphenyl)-methyl]-2(5H)-furanon:

Das Gemisch aus 12.7 g (50 mmol) threo-5-(2-Chlorphenylhydroxymethyl)-4-methoxy-2(5H)-furanon, 100 ml Dimethoxymethan, 1.7 g (20 mmol) Lithiumbromid und 0.95 g (5 mmol) p-Toluolsulfonsäure-Hydrat wird 4 Tage unter Rühren zum Sieden unter Rückfluß erhitzt. Man evaporiert, versetzt mit 50 ml Dichlormethan, wäscht mit Wasser frei von Lithiumbromid und p-Toluolsulfonsäure, evaporiert die organische Phase und kristallisiert das Rohprodukt 2 mal aus Methanol um. Man erhält 10.68 g (35.75 mmol) des Methoxymethylethers mit Schmp. 141 - 143°C.
Ausbeute: 71.7 %
Analyse: $C_{14}H_{15}ClO_5$ (298.73)
Ber.: C (56.29), H (5.06), Cl (11.87)
Gef.: C (56.52), H (5.10), Cl (11.9)
300 MHz-$^1$NMR: 7.3-7.6 (4 H, m, aromat. Protonen),
5.47 (1 H, d, $J_{H-3/H-5}$ = 0.88 Hz, H-3)
5.32 (1 H, d, $H_{H\alpha}$/H-5 = 2.65 Hz, H=α)
5.09 Hz (1 H, dd, H-5),
4.55 und 4.42 (2 H, AB-System, $J_{AB}$ = 7.08 Hz, O-$CH_2$-O),
3.94 (3 H, s, 4-$OCH_3$),
3.20 (3 H, s, ω-$OCH_3$)

Beispiel Nr. 108

threo-4-Methoxy-5-[methoxyethoxymethoxy-(2-chlorphenyl)-methyl]-2(5H)-furanon:

Die Lösung von 10.2 g (40 mmol) threo-5-(2-Chlorphenylhydroxymethyl)-4-methoxy-2(5H)-furanon in 250 ml Dichlormethan wird nacheinander mit 27 ml (238 mmol) Methoxyethoxymethylchlorid und 40 ml (234 mmol) N-Ethyldiisopropylamin versetzt und 30 Stdn. zum Sieden unter Rückfluß erhitzt. Nach dem Abkühlen wäscht man mit insgesamt 260 ml 1M-Salzsäure und mit 2 x 50 ml Wasser, trocknet über wasserfreiem Natriumsulfat, evaporiert und kristallisiert das ölige Rohprodukt 2 mal aus Ethanol um. Man erhält 9.86 g (28.8 mmol) des Methoxyethoxymethylethers mit Schmp. 102 - 104°C.
Ausbeute: 72 %
Analyse: $C_{16}H_{19}ClO_6$ (342.78)
Ber.: C (56.07), H (5.59), Cl (10.34)
Gef.: C (55.72), H (5.60), Cl (10.2)

Beispiele Nr. 109 bis 124:

Nach den in Beispielen Nr. 106 bis 108 beschriebenen Methoden zur Veretherung und Umacetalisierung werden die in Tabelle 12 aufgeführten Ether- und Acetal-Derivate der zuvor beschriebenen threo-4-Alkoxy-5-[(subst.)phenylhydroxymethyl]-2(5H)-furanone hergestellt. Die analytischen Daten sind in Tabelle 13 zusammengefaßt.

Tabelle 12: Ether- und Acetal-Derivate

| Beispiel Nr. | Bezeichnung | Schmp. [°C] (umkrist.aus) |
|---|---|---|
| 109 | threo-4-Methoxy-5-[methoxy-(phenyl)-methyl]-2(5H)-furanon | 91-95 (Ether/Heptan) |
| 110 | threo-4-Methoxy-5-[methoxy-(2-chlorphenyl)-methyl]-2(5H)-furanon | 118-120 (Ether/Pentan) |
| 111 | threo-4-Methoxy-5-[methoxy-(2.4-dichlorphenyl)-methyl]-2(5H)-furanon | 124-125 (Ether / Petrolether) |
| 112 | threo-4-Methoxy-5-[methoxy-(2.5-dichlorphenyl)-methyl]-2(5H)-furanon | 95 (Ether / Petrolether) |
| 113 | threo-4-Methoxy-5-[methoxymethoxy-(phenyl)-methyl]-2(5H)-furanon | 93-96 (Methanol) |
| 114 | threo-4-Methoxy-5-[methoxymethoxy-(2.4-dichlorphenyl)-methyl]-2(5H)-furanon | 142-145 (Methanol) |
| 115 | threo-4-Methoxy-5-[methoxymethoxy-(2-fluorphenyl)-methyl]-2(5H)-furanon | 102-104 (Methanol) |
| 116 | threo-4-Methoxy-5[methoxymethoxy-(2-methylphenyl)-methyl]-2(5H)-furanon | 141-144 (Ethylacetat) |
| 117 | threo-4-Methoxy-5-(methoxymethoxy-(4-bromphenyl)-methyl]-2(5H)-furanon | 144-146 (Ethanol) |
| 118 | threo-4-Methoxy-5-[methoxymethoxy-(2-bromphenyl)-methyl]-2(5H)-furanon | 160-161 (Ethanol) |
| 119 | threo-4-Methoxy-5[methoxymethoxy-(4-chlorphenyl)-methyl]-2(5H)-furanon | 130 (Methanol) |

Fortsetzung Tabelle 12

| Beispiel Nr. | Bezeichnung | Schmp. [°C] (umkrist.aus) |
|---|---|---|
| 120 | threo-4-Methoxy-5-[methoxyethoxy-methoxy-(2.4-dichlorphenyl)-methyl]-2(5H)-furanon | 104-107 (Ethanol) |
| 121 | threo-5-[1',2'-Dimethoxyethoxy-(2-chlorphenyl)-methyl]-4-methoxy-2(5H)-furanon | 131-134 (Ethanol) |
| 122 | threo-5-[1'-Ethoxypropoxy-(2-chlorphenyl)-methyl]-4-methoxy-2-(5H)-furanon | 106-108 (Ether / Pentan) |
| 123 | threo-4-Methoxy-5-[methoxy-(2-fluorphenyl)-methyl]-2(5H)-furanon | 106-108 (Ether) |
| 124 | threo-4-Methoxy-5-[methoxy-(2-bromphenyl)-methyl]-2(5H)-furanon | 123-125 (Ether / Methanol) |

Tabelle 13: Elementaranalysen

| Beispiel Nr. | Summenformel / Molmasse | % Ber. C / % Gef. C | H / H | Sonstige / Sonstige |
|---|---|---|---|---|
| 109 | $C_{13}H_{14}O_4$ | 66.66 | 6.02 | |
| | 234.25 | 66.84 | 6.25 | |
| 110 | $C_{13}H_{13}ClO_5$ | 58.11 | 4.88 | Cl (13.19) |
| | 268.69 | 58.02 | 5.01 | 13.5 |
| 111 | $C_{13}H_{12}Cl_2O_5$ | 51.50 | 3.99 | Cl (23.39) |
| | 303.15 | 51.56 | 3.97 | 23.5 |
| 112 | $C_{13}H_{12}Cl_2O_5$ | 51.50 | 3.99 | Cl (23.39) |
| | 303.15 | 52.02 | 4.16 | 23.4 |
| 113 | $C_{14}H_{16}O_5$ | 63.63 | 6.10 | |
| | 264.29 | 63.65 | 6.09 | |
| 114 | $C_{14}H_{14}Cl_2O_5$ | 50.47 | 4.23 | Cl (21.28) |
| | 333.18 | 50.36 | 4.22 | 20.9 |
| 115 | $C_{14}H_{15}FO_5$ | 59.57 | 5.36 | F (6.7) |
| | 282.28 | 59.32 | 5.49 | 7.3 |
| 116 | $C_{15}H_{18}O_5$ | 64.74 | 6.52 | |
| | 278.31 | 64.71 | 6.71 | |
| 117 | $C_{14}H_{15}BrO_5$ | 49.00 | 4.41 | Br (23.28) |
| | 343.19 | 48.44 | 4.36 | 22.3 |
| 118 | $C_{14}H_{15}BrO_5$ | 49.00 | 4.41 | Br (23.28) |
| | 343.19 | 48.38 | 4.27 | 23.2 |
| 119 | $C_{14}H_{15}ClO_5$ | 56.29 | 5.06 | Cl (11.87) |
| | 298.73 | 55.47 | 5.00 | 11.8 |
| 120 | $C_{16}H_{18}Cl_2O_6$ | 50.95 | 4.81 | Cl (18.20) |
| | 377.23 | 50.62 | 4.76 | 18.2 |
| 121 | $C_{16}H_{19}ClO_6$ | 56.07 | 5.59 | Cl (10.34) |
| | 342.78 | 55.81 | 5.47 | 10.3 |
| 122 | $C_{17}H_{21}ClO_5$ | 59.91 | 6.21 | Cl (10.40) |
| | 340.81 | 59.62 | 6.10 | 10.6 |
| 123 | $C_{13}H_{13}FO_4$ | 61.90 | 5.19 | F (7.53) |
| | 252.24 | 61.75 | 5.16 | |
| 124 | $C_{13}H_{13}BrO_4$ | 49.86 | 4.18 | Br (25.52) |
| | 313.15 | 49.84 | 4.18 | 25.2 |

Zur Ermittlung der anticonvulsiven / antiepileptischen Aktivität der erfindungsgemäßen Substanzen wurde die von E.A. Swinyard et al., J. Pharmacol.exp.Therapeut. 106, 319-330 (1952) und von L.A. Woodbury et. al., Arch. int. Pharacodyn. 92, 97-107 (1952) beschriebene Methodik verwendet. Männlichen Mäusen (NMRI) mit einem Körpergewicht von 20 - 25 g wird über Corneal-Elektroden ein Wechselstrom von 50 Hz und 50 mA während 0.2 sec. appliziert (HSE-Schock-Reizgerät; Typ 207). Der Maximal-Elektroschock-Krampf (MES) besteht aus einer tonischen Streckung der Hinterextremitäten, klonischen Zuckungen und einem Bewußtseinverlust. Als Wirksamkeitskriterium wird die Hemmung des Extensorenkrampfes durch die erfindungsgemäßen Substanzen betrachtet. Die Mäuse hatten vor den Experimenten freien Zugang zu Futter und Wasser. Die Testsubstanzen wurden als Suspension in 0.2% Agar p.o. durch Schlundsonde appliziert; die Kontrolltiere erhielten adäquate Volumina Agar. 1 Stunde nach Applikation wurde der Test auf Schutzwirkung gegen MES durchgeführt.

In Tabelle 14 sind die Ergebnisse des MES-Tests bei Dosierungen von 50 bis 150 mg der erfindungsgemäßen Verbindungen pro kg Körpergewicht im Vergleich zu gebräuchlichen Antiepileptika aufgeführt. Als % Wirkung ist der prozentuale Anteil derjenigen Tiere angegeben, die bei dem MES-Test zu 100% gegen den Extensorenkrampf geschützt waren.

In Tabelle 15 sind als ED 50-Werte diejenigen Dosierungen der erfindungsgemäßen Substanzen und von gebräuchlichen Antiepileptika aufgeführt, die im MES-Test 1 Stunde nach Applikation 50% der Tiere vollständig vor dem Extensorenkrampf zu schützen vermochten. Die Bestimmung der ED 50-Werte und der Vertrauensgrenzen (5% Irrtumswahrscheinlichkeit) erfolgte nach Lichtfield und Wilcoxon [J. Pharmacol.exp. Therapeut. 96, 99 (1949)] jeweils mit 4 bis 5 Tiergruppen zu 8 - 10 Tieren je Dosisstufe.

Während der oben beschriebenen Experimente wurden die Tiere innerhalb der gesamten Versuchsdauer (bis zu 4 Stunden) auf Anzeichen substanzbedingter Verhaltensänderungen und Neurotoxizität (Motilität, Muskeltonus, Atemfrequenz, Körpertempertur und Allgemeinverhalten) beobachtet. Bis zu einer Dosierung von 100 mg/kg konnten bei allen geprüften erfindungsgemäßen Substanzen keinerlei Neurotoxizitäts-Symptome festgestellt werden. Im Vergleich hierzu sind in Tabelle 16 die unteren Grenzdosierungen gebräuchlicher Anticonvulsiva angeführt, die nach p.o. Applikation Neurotoxizitätssymptome bei Mäusen hervorrufen.

Bei sämtlichen erfindungsgemäßen Verbindungen wurde in Dosierungen bis zu 300 mg/kg p.o. keinerlei Mortalität der Mäuse festgestellt. Orientierende toxikologische Untersuchungen der Substanzen gemäß Beispielen Nr. 64, 65, 79 und 81 an Ratten zeigten bis zur Dosierung von 1000 mg/kg p.o. keinerlei Mortalität der Tiere.

Die vorliegenden Untersuchungen zeigen im Ergebnis eine gute antikonvulsive Wirkung und hervorragende therapeutische Breite der erfindungsgemäßen Verbindungen.

Tabelle 14: Ergebnisse des MES-Tests

| Substanz gemäß Beisp.Nr. | Dosis [mg/kg] | Anzahl der Tiere im Versuch | Schutzwirkung [%] nach 1 Stde. |
|---|---|---|---|
| 64 | 100 | 8 | 100 |
| 65 | 100 | 24 | 79 |
| 66 | 100 | 10 | 100 |
| 67 | 100 | 8 | 75 |
| 68 | 100 | 10 | 100 |
| 69 | 100 | 10 | 70 |
| 70 | 100 | 10 | 100 |
| 71 | 100 | 10 | 100 |
| 72 | 100 | 10 | 80 |
| 73 | 100 | 10 | 60 |
| 74 | 100 | 10 | 100 |
| 79 | 100 | 10 | 60 |
| 80 | 100 | 10 | 90 |
| 81 | 100 | 10 | 100 |
| 82 | 150 | 10 | 90 |
| 83 | 100 | 18 | 50 |
| 84 | 100 | 10 | 70 |
| 86 | 50 | 10 | 100 |
| 87 | 100 | 10 | 40 |
| 88 | 100 | 10 | 80 |
| 89 | 100 | 10 | 100 |
| 93 | 100 | 8 | 37.5 |
| 96 | 100 | 8 | 62.5 |
| 98 | 100 | 8 | 25 |
| 99 | 100 | 10 | 90 |
| 100 | 100 | 10 | 100 |
| 102 | 100 | 8 | 62.5 |
| 103 | 100 | 8 | 100 |
| 104 | 100 | 8 | 37.5 |
| 105 | 100 | 8 | 12.5 |
| 106 | 100 | 10 | 30 |
| 107 | 100 | 10 | 100 |

Fortsetzung Tabelle 14: MES-Test

| Substanz gemäß Beisp.Nr. | Dosis [mg/kg] | Anzahl der Tiere im Versuch | Schutzwirkung [%] nach 1 Stde. |
|---|---|---|---|
| 108 | 100 | 10 | 70 |
| 109 | 100 | 10 | 40 |
| 110 | 100 | 10 | 100 |
| 111 | 100 | 10 | 100 |
| 112 | 100 | 10 | 100 |
| 113 | 100 | 10 | 100 |
| 114 | 100 | 10 | 10 |
| 115 | 100 | 10 | 100 |
| 116 | 100 | 10 | 70 |
| 118 | 100 | 10 | 100 |
| 121 | 50 | 10 | 70 |
| 122 | 50 | 10 | 100 |
| 123 | 50 | 10 | 100 |
| 124 | 50 | 10 | 100 |
| Carbamazepin | 50 | 10 | 100 |
| Diazepam | 50 | 10 | 100 |
| Diphenyl-hydantoin | 50 | 10 | 100 |
| Ethosuximid | 100 | 10 | 0 |
| Phenobarbi-tal | 100 | 10 | 100 |
| Valproin-säure | 100 | 10 | 0 |

Tabelle 15: ED 50-Werte im MES-Test

| Substanz gemäß Beisp.Nr. | Anzahl der Tiere | ED-50 [mg / kg] | Vertrauensgrenze [mg / kg] |
|---|---|---|---|
| 64 | 50 | 19.75 | (17.10 - 22.81) |
| 65 | 60 | 69.0 | (63.07 - 75.49) |
| 67 | 60 | 73.5 | (63.86 - 84.60) |
| 68 | 60 | 23.5 | (23.38 - 27.10) |
| 69 | 50 | 93 | (84.69 - 102.10) |
| 70 | 60 | 22.0 | (19.08 - 25.37) |
| 79 | 60 | 82.5 | (75.61 - 90.01) |
| 81 | 40 | 19.5 | (13.47 - 28.24) |
| 96 | 60 | 74.0 | (63.46 - 86.28) |
| 98 | 60 | 84.5 | (78.24 - 91.26) |
| 100 | 60 | 67.0 | (60.31 - 74.44) |
| 103 | 60 | 37.0 | (28.70 - 47.50) |
| 107 | 60 | 24.5 | (22.15 - 27.10) |
| 110 | 60 | 27.5 | (25.45 - 29.71) |
| Carbamazepin | 60 | 14.6 | (12.36 - 17.24) |
| Diazepam | 40 | 13.8 | ( 9.93 - 19.18) |
| Diphenylhydantoin(4 h) | 40 | 9.1 | ( 7.23 - 11.47) |
| Ethosuximid | 50 | >250 | |
| Pentobarbital (s.c.) | 60 | 35.2 | (31.9 - 38.8) |
| Phenobarbital | 50 | 19.2 | (16.29 - 22.61) |
| Valproinsäure (i.p.) | 50 | 205 | (169 - 248) |

Tabelle 16: Neurotoxische Grenzdosis an der Maus

| Substanz | Dosis p.o. [mg / kg] |
|---|---|
| Carbamazepin | 100 |
| Diazepam | 40 |
| Diphenylhydantoin | 100 |
| Ethosuximid | 500 |
| Pentobarbital | 60 |
| Phenobarbital | 60 |
| Valproinsäure | 350 |
| erfindungsgemäße Verbindungen gemäß Beispielen Nr. 64 - 124 | alle >100 |

Beispiele für die Herstellung pharmazeutischer Zubereitungen der erfindungsgemäßen Substanzen.

A. Tabletten:

Zur Herstellung von Tabletten a 250 mg Einzelgewicht, die je nach gewünschter Wirkungsstärke 5 bis 100 mg Wirkstoff enthalten, benötigt man
erfindungsgemäße Substanz 200 bis 4000 g
Zellulosepulver 2000 g
Maisstärke 1200 g
kolloide Kieselsäure 80 g
Magnesiumstearat 20 g
Milchzucker ad 10 000 g
Wirkstoff und Hilfsstoffe werden homogen vermischt und in der üblichen Weise zu Tabletten von je 250 mg Gewicht und 9 mm Durchmesser verpreßt. Falls gewünscht, werden die Tabletten mit einem Filmüberzug versehen.

B. Kapseln:

Zur Herstellung von Kapseln, die je nach gewünschter Wirkungsstärke 5 bis 100 mg Wirkstoff enthalten, benötigt man erfindungsgemäße Substanz 500 bis 10 000 g
Maisstärke 2000 g
kolloide Kieselsäure 300 g
Magnesiumstearat 50 g
Zellulosepulver ad 20 000 g
Die feingepulverten Stoffe werden homogen gemischt und in Hartgelatinekapseln der Größe 2 in der Menge von 200 mg pro Kapsel abgefüllt.

C. Saft:

Zur Herstellung eines Saftes mit einem Gehalt von 0.035 bis 0.7 Gew.-% Wirkstoff, je nach gewünschter Wirkungsstärke, benötigt man
erfindungsgemäße Substanz 35 bis 700 g
Propylenglykol 20 000 g
Glycerin 20 000 g
Methylzellulose 1000 g

Natriumcyclamat 500 g
Saccharin-Natrium 50 g
demineralisiertes Wasser ad 100 000 g
Der Wirkstoff wird fein gemahlen und homogen in die Lösung der Hilfsstoffe eingerührt.

D. Zäpfchen:

Zur Herstellung von Zäpfchen a 3 g Einzelgewicht, enthaltend 5 bis 100 mg Wirkstoff pro Zäpfchen, je nach gewünschter Wirkungsstärke, benötigt man
erfindungsgemäße Substanz 50 bis 1000 g
kolloide Kieselsäure 60 g
Lecithin 150 g
Hartfett ad 30 000 g
Der feingemahlene Wirkstoff wird in die Schmelze der Hilfsstoffe homogen eingerührt und zu Zäpfchen von 3 g Eigengewicht vergossen.

**Patentansprüche**

1. 5-Arylalkyl-4-alkoxy-2(5H)-furanone der allgemeinen Formel I

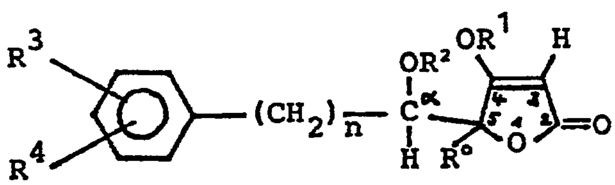

(I)

worin die Sauerstoffatome an C-5 und C-α relativ zueinander die threo-Position einnehmen und worin
n die Zahl 0, 1 oder 2,
$R^0$ ein Wasserstoffatom oder eine Niederalkylgruppe mit 1 bis 3 C-Atomen,
$R^1$ eine geradkettige oder verzweigte Alkylgruppe mit 1 bis 5 C-Atomen,
$R^2$ ein Wasserstoffatom, eine Niederalkylgruppe mit 1 bis 3 C-Atomen oder den Rest

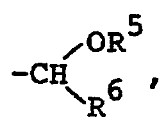

in welchem
$R^5$ eine Niederalkylgruppe mit 1 bis 5 C-Atomen, die Ethoxyethyl- oder Methoxyethylgruppe und
$R^6$ ein Wasserstoffatom, eine Niederalkylgruppe mit 1 bis 5 C-Atomen oder die Methoxymethylgruppe bedeuten,
$R^3$ und $R^4$ unabhängig voneinander ein Wasserstoff-, Fluor-, Chlor- oder Bromatom, eine Niederalkylgruppe mit 1 bis 3 C-Atomen, eine Perfluorniederalkylgruppe mit 1 bis 3 C-Atomen, die Difluormethoxy- oder Nitrogruppe
bedeuten, unter Ausschluß derjenigen Verbindungen der allgemeinen Formel I, worin
$R^2$ H oder $CH_3$ bedeutet, wenn
$n = 0$ oder 2, $R^0 = H$, $R^1 = CH_3$, $R^3 = H$ und $R^4 = H$ ist.

2. 4-Methoxy-5-phenylhydroxymethyl-2(5H)-furanone der allgemeinen Formel II

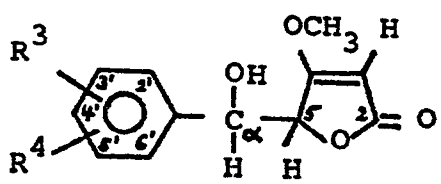

(II)

worin die Sauerstoffatome an C-5 und C-α relativ zueinander die threo-Position einnehmen und worin einer der beiden Reste $R^3$ und $R^4$ ein Wasserstoffatom und der andere ein in 2'-Stellung befindliches Fluor-, Chlor- oder Bromatom oder eine in 2'-Stellung befindliche Methyl-, Trifluormethyl- oder Nitrogruppe bedeuten.

3. 4-Methoxy-5-phenylhydroxymethyl-2(5H)-furanone der allgemeinen Formel II, worin die Sauerstoffatome an C-5 und C-α relativ zueinander die threo-Position einnehmen und worin einer der beiden Reste $R^3$ und $R^4$
- ein Fluor-, Chlor- oder Bromatom oder eine Trifluormethylgruppe, jeweils in 2'-Stellung,
und der andere
- ein Chlor- oder Bromatom oder eine Trifluormethylgruppe, jeweils in 4'-, 5'- oder 6'-Stellung,
bedeuten.

4. 4-Methoxy-5-[methoxy-(phenyl)-methyl]-2(5H)-furanone der allgemeinen Formel III

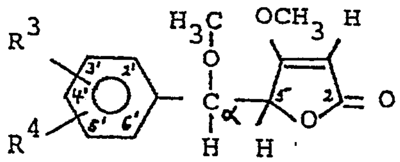

(III)

worin die Sauerstoffatome an C-5 und C-α relativ zueinander die threo-Position einnehmen und worin $R^3$ und $R^4$ die in Anspruch 1 angegebene Bedeutung besitzen, unter Ausschluß derjenigen Verbindungen der allgemeinen Formel III, worin beide Reste $R^3$ und $R^4$ jeweils ein Wasserstoffatom bedeuten.

5. 4-Methoxy-5-phenylmethyl-2(5H)-furanone der allgemeinen Formel IV

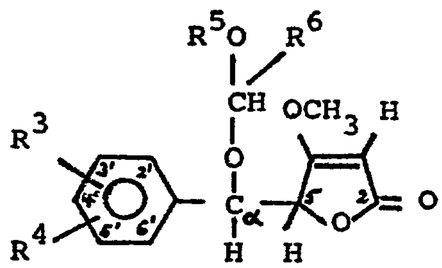

(IV)

worin die Sauerstoffatome an C-5 und C-α relativ zueinander die threo-Position einnehmen und worin
$R^5$ eine Methyl- oder Methoxyethylgruppe und
$R^3$, $R^4$ und $R^6$ die in Anspruch 1 angegebene Bedeutung besitzen.

6. threo-5-(2'-Chlorphenylhydroxymethyl)-4-methoxy-2(5H)-furanon.
7. threo-5-(2'-Bromphenylhydroxymethyl)-4-methoxy-2(5H)-furanon;
8. threo-5-(2'-Fluorphenylhydroxymethyl)-4-methoxy-2(5H)-furanon.
9. threo-5-(2'-Trifluormethyl-phenyl-hydroxymethyl)-4-methoxy-2(5H)-furanon;
10. threo-5-(2',5'-Dichlorphenyl)-4-methoxy-2(5H)-furanon.
11. threo-5-(2',4'-Dichlorphenyl)-4-methoxy-2(5H)-furanon;
12. threo-4-Methoxy-5-[methoxymethoxy-(2'-chlorphenyl)-methyl]-2(5H)-furanon.
13. threo-4-Methoxy-5-[methoxymethoxy-(2'-bromphenyl)-methyl]-2(5H)-furanon;
14. threo-4-Methoxy-5-[methoxymethoxy-(2'-fluorphenyl)-methyl]-2(5H)-furanon;
15. threo-4-Methoxy-5-[methoxymethoxy-(2'-trifluormethyl-phenyl)-methyl]-2(5H)-furanon;
16. threo-4-Methoxy-5-[methoxy-(2'-chlorphenyl)-methyl]-2(5H)-furanon.
17. 3-Alkoxy-5-(subst.)phenyl-2(E),4(E)-pentadienoate der allgemeinen Formel IX

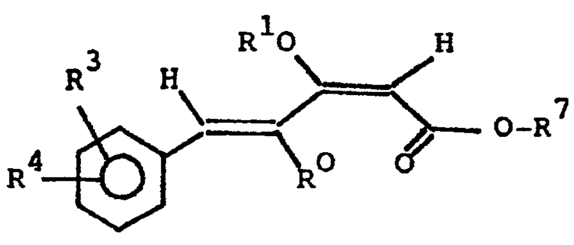

(IX)·

worin
$R^0$, $R^1$, $R^3$ und $R^4$ die in Anspruch 1 angegebenen Bedeutungen besitzen und
$R^7$ ein Wasserstoffatom, eine geradkettige oder verzweigte Alkylgruppe mit 1 bis 10 C-Atomen oder eine Aralkylgruppe mit 7 bis 10 C-Atomen,
ist, mit Ausnahme derjenigen Verbindung, worin $R^0$ = H, $R^1$ = Ethyl, $R^3$ = H, $R^4$ = H und $R^7$ = Ethyl.

18. Verbindungen nach Anspruch 17, dadurch gekennzeichnet, dass $R^7$ ein Wasserstoffatom, eine geradkettige oder verzweigte Alkylgruppe mit 1 bis 4 C-Atomen oder eine Benzylgruppe ist.

19. Verfahren zur Herstellung der Verbindungen gemäss Anspruch 1 mit der allgemeinen Formel I, worin n = 0 ist, gekennzeichnet durch die Kombination folgender Verfahrensstufen:
(A) Ein Benzaldehyd der allgemeinen Formel VI

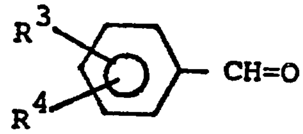

(VI)

worin $R^3$ und $R^4$ die im Zusammenhang mit der Erläuterung der Formel I genannten Bedeutungen besitzen, wird mit einem 3-Alkoxy-2(E)-alkensäure-niederalkylester der allgemeinen Formel VII

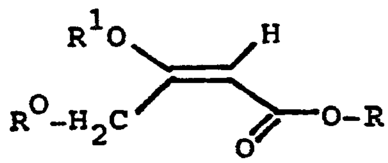

(VII)

worin $R^0$ und $R^1$ die im Zusammenhang mit der Erläuterung der allgemeinen Formel I genannten Bedeutungen besitzen und R eine Niedrigalkylgruppe mit 1 bis 4 C-Atomen ist, unter hydrolytischer Abspaltung des Restes R stereoselektiv zur entsprechend substituierten 2(E),4(E)-Pentadiensäure der allgemeinen Formel VIII

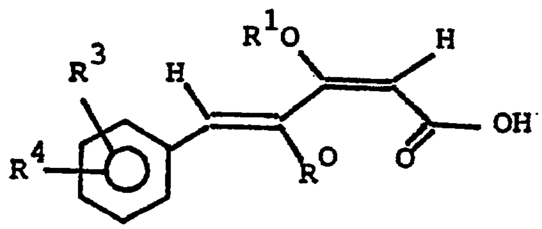

(VIII)

worin $R^0$, $R^1$, $R^3$ und $R^4$ die genannten Bedeutungen besitzen, kondensiert;
(B) die so entstandene Pentadiensäure wird in einen Ester der allgemeinen Formel IX

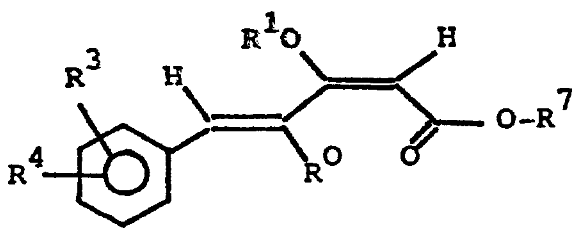

(IX)

worin $R^7$ eine geradkettige oder verzweigte Alkylgruppe mit 1 bis 10 C-Atomen oder eine Aralkylgruppe mit 7 bis 10 C-Atomen ist, übergeführt;
(C) der Ester der allgemeinen Formel IX wird durch cis-Dihydroxylierung mittels katalytischer Mengen $OsO_4$ und eines Oxidationsmittels zum entsprechenden 3-Alkoxy-4,5(threo)-dihydroxy-5-($R^3$,$R^4$-subst.)phenyl-2(E)-pentensäureester der allgemeinen Formel XI umgesetzt

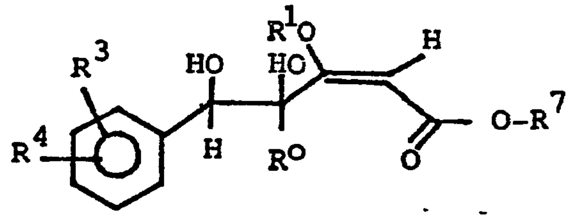

(XI)

(D) woraus unter 1.4-Eliminierung des Alkohols $R^7OH$ das entsprechende threo-4-Alkoxy-5-[($R^3$,$R^4$-subst.)phenylhydroxymethyl]-2(5H)-furanon der allgemeinen Formel I mit $R^2$ = H gebildet wird,
(E) welches anschließend gegebenenfalls mit einer reaktiven Verbindung der allgemeinen Formel

$R^2$-X, worin $R^2$ die im Zusammenhang mit der Erläuterung der allgemeinen Formel I genannte Bedeutung, mit Ausnahme von Wasserstoff, besitzt und X eine reaktive Gruppe ist, z.B. Halogen oder Alkoxy, unter Abspaltung von X-H zur entsprechenden Verbindung der allgemeinen Formel I mit $R^2 \neq H$ umgesetzt wird.

20. Verfahren nach Anspruch 19, dadurch gekennzeichnet, daß die in Stufe (A) gebildete Pentadiensäure der allgemeinen Formel VIII der cis-Dihydroxylierung gemäß Stufe (C) unter Bildung der entsprechend substituierten Pentensäure der allgemeinen Formel XI mit $R^7$ = H unterworfen wird, wonach die Pentensäure gemäß Stufe (D) unter 1.4-Eliminierung von $H_2O$ zum entsprechend substituierten Furanon der allgemeinen Formel I mit $R^2$ = H umgesetzt wird, welches anschließend gegebenenfalls gemäß Stufe (E) zu einer Verbindung der allgemeinen Formel I mit $R^2 \neq H$ umgesetzt wird.

21. Verfahren nach Anspruch 19 oder 20, dadurch gekennzeichnet, daß die Kondensation der Stufe (A) in einem dipolaren aprotischen Lösungsmittel und in basischem Milieu durchgeführt wird.

22. Verfahren nach Anspruch 21, dadurch gekennzeichnet, daß die Kondensation in Dimethylsulfoxid unter Zugabe von Alkalimetallhydroxid, quartären Ammoniumbasen oder deren Gemischen durchgeführt wird.

23. Verfahren nach einem der Ansprüche 19 bis 22, dadurch gekennzeichnet, daß die Kondensation unter Inertgasatmosphäre bei einer Temperatur zwischen etwa 70 und 140°C durchgeführt wird.

24. Verfahren nach einem der Ansprüche 19 bis 23, dadurch gekennzeichnet, daß in Stufe (C) Alkylhydroperoxide, tertiäre Amin-N-oxide oder Chlorate als Oxidationsmittel verwendet werden.

25. Verfahren nach Anspruch 24, dadurch gekennzeichnet, daß die Dihydroxylierung in Stufe (C) in Gegenwart einer quartären Ammoniumbase oder eines quartären Ammoniumsalzes durchgeführt wird.

26. Arzneimittel, enthaltend gegebenenfalls zusammen mit einem pharmakologisch inerten Excipiens eine oder mehrere der Verbindungen der allgemeinen Formel I, einschließlich derjenigen Verbindungen der allgemeinen Formel I, worin $R^2$ H oder $CH_3$ bedeutet, wenn n = 0 oder 2, $R^0$ = H, $R^1$ = $CH_3$, $R^3$ = H und $R^4$ = H ist.

27. Verwendung der Verbindungen der allgemeinen Formel I, einschliesslich derjenigen, worin $R^2$ H oder $CH_3$ bedeutet, wenn n = 0 oder 2, $R^0$ = H, $R^1$ = $CH_3$, $R^3$ = H und $R^4$ = H ist, und IX, einschliesslich derjenigen, worin $R^0$ = H, $R^1$ = Ethyl, $R^3$ = H, $R^4$ = H und $R^7$ = Ethyl bedeuten, bei der Herstellung von Antikonvulsiva und Antiepileptika.

## Claims

1. 5-Arylalkyl-4-alkoxy-2(5H)-furanones of the general formula I

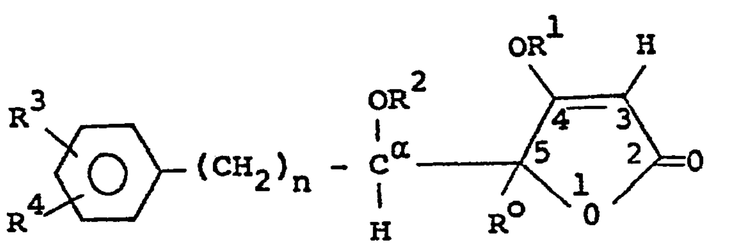

(I)

wherein the oxygen atoms on C-5 and C-α, relative to one another, are in the threo-position and wherein
n is 0, 1 or 2,
$R^0$ is a hydrogen atom or a lower alkyl radical having 1 to 3 C-atoms,
$R^1$ is a straight-chained or branched alkyl radical having 1 to 5 C-atoms,
$R^2$ is a hydrogen atom, a lower alkyl radical having 1 to 3 C-atoms or the radical

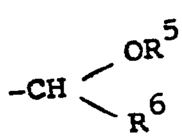

,

wherein
$R^5$ is a lower alkyl radical having 1 to 5 C-atoms, ethoxyethyl or methoxyethyl, and
$R^6$ is a hydrogen atom, a lower alkyl radical having 1 to 5 C-atoms or methoxymethyl,
$R^3$ and $R^4$ independently of one another, each are a hydrogen, fluorine, chlorine or bromine atom, a lower alkyl radical having 1 to 3 C-atoms, a perfluoroalkyl radical having 1 to 3 C-atoms, a difluoromethoxy radical or a nitro group with the exclusion of those compounds of general formula I, wherein
$R^2$ is H or $CH_3$ when
n = 0 or 2, $R^0$ = H, $R^1$ = $CH_3$, $R^3$ = H and $R^4$ = H.

2. 4-Methoxy-5-phenylhydroxymethyl-2(5H)-furanones of the general formula II

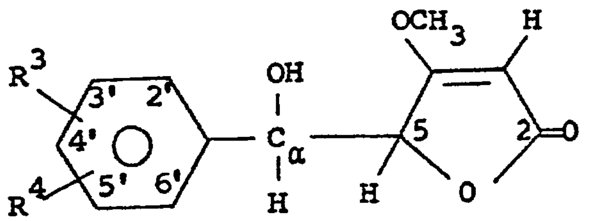

(II)

wherein the oxygen atoms on C-5 and C-α, relative to one another, are in the threo-position and wherein one of the two symbols $R^3$ and $R^4$ signifies a hydrogen atom and the other a fluorine, chlorine or bromine atom present in the 2′-position or a methyl, trifluoromethyl or nitro group present in the 2′-position.

3. 4-Methoxy-5-phenylhydroxymethyl-2(5H)-furanones of general formula II, wherein the oxygen atoms on C-5 and C-α, relative to one another, are in the threo-position and wherein one of the two symbols $R^3$ and $R^4$ signifies

– a fluorine, chlorine or bromine atom or a trifluoromethyl radical, in each case in the 2′-position, and the other signifies

– a chlorine or bromine atom or a trifluoromethyl radical, in each case in the 4′-, 5′- or 6′-position.

4. 4-Methoxy-5-[methoxy-(phenyl)-methyl]-2(5H)-furanones of the general formula III

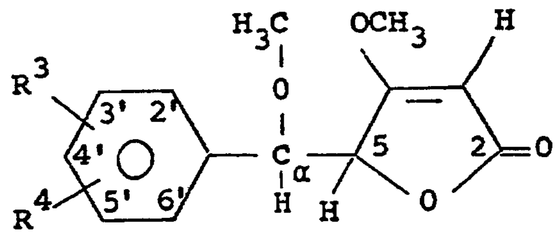

(III)

wherein the oxygen atoms on C-5 and C-α, relative to one another, are in the threo-position and wherein $R^3$ and $R^4$ have the same meanings as in claim 1, with the exclusion of those compounds of general formula III wherein both symbols $R^3$ and $R^4$ each represent hydrogen atoms.

5. 4-Methoxy-5-phenylmethyl-2(5H)-furanones of the general formula IV

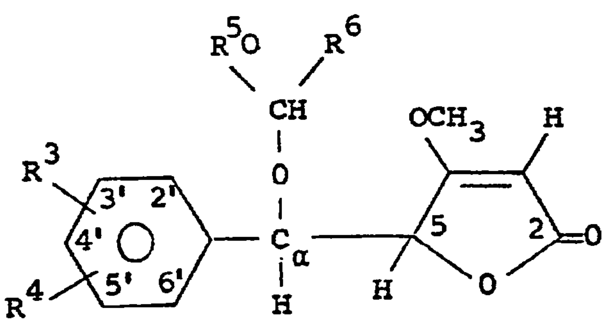

(IV)

wherein the oxygen atoms on C-5 and C-α, relative to one another, are in the threo-position and wherein $R^5$ is a methyl or methoxyethyl radical and
$R^3$, $R^4$ and $R^6$ have the meanings given in claim 1.

6. threo-5-(2′-Chlorophenylhydroxymethyl)-4-methoxy-2(5H)-furanone.
7. threo-5-(2′-Bromophenylhydroxymethyl)-4-methoxy-2(5H)-furanone.
8. threo-5-(2′-Fluorophenylhydroxymethyl)-4-methoxy-2(5H)-furanone.
9. threo-5-(2′-Trifluoromethylphenylhydroxymethyl)-4-methoxy-2(5H)-furanone.
10. threo-5-(2′,5′-Dichlorophenylhydroxymethyl)-4-methoxy-2(5H)-furanone.
11. threo-5-(2′,4′-Dichlorophenylhydroxymethyl-4-methoxy-2(5H)-furanone.
12. threo-4-Methoxy-5-[methoxymethoxy-(2′-chlorophenyl)-methyl]-2(5H)-furanone.
13. threo-4-Methoxy-5-[methoxymethoxy-(2′-bromophenyl)-methyl]-2(5H)-furanone.
14. threo-4-Methoxy-5-[methoxymethoxy-(2′-fluorophenyl)-methyl] -2(5H)-furanone.
15. threo-4-Methoxy-5-[methoxymethoxy-(2′-trifluoromethylphenyl)-methyl]-2(5H)-furanone.
16. threo-4-Methoxy-5-[methoxy-(2′-chlorophenyl)-methyl]-2(5H)-furanone.
17. 3-Alkoxy-5-(subst.)-phenyl-2(E), 4(E)-pentadienoates of the general formula IX

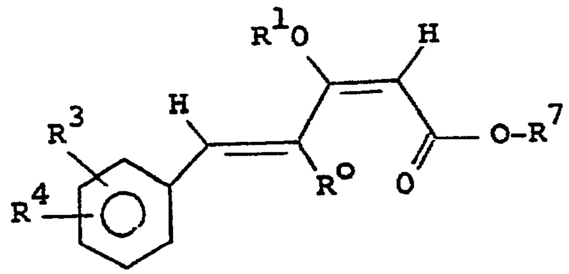

(IX)

wherein
$R^0$, $R^1$, $R^3$ and $R^4$ have the meanings given in claim 1 and
$R^7$ is a hydrogen atom, a straight-chained or branched alkyl radical having 1 to 10 C-atoms or an aralkyl radical having 7 to 10 C-atoms,
with the exception of those compounds wherein $R^0$ = H, $R^1$ = ethyl, $R^3$ = H, $R^4$ = H and $R^7$ = ethyl.

18. Compounds according to claim 17, characterized in that $R^7$ is a hydrogen atom, a straight-chained or branched alkyl radical having 1 to 4 C-atoms or a benzyl radical.

19. Process for the preparation of the compounds according to claim 1 of general formula I, wherein n = 0, characterized by a combination of the following process steps:

(A) a benzaldehyde of the general formula VI

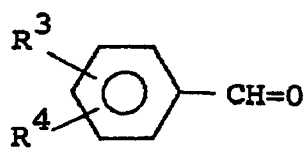

(VI)

wherein $R^3$ and $R^4$ have the same meanings as explained in connection with general formula I, is condensed with a 3-alkoxy-2(E)-alkenoic acid lower alkyl ester of the general formula VII

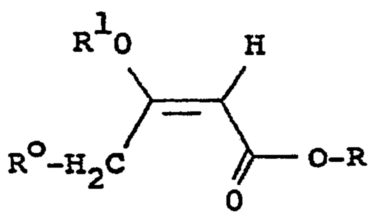

(VII)

wherein $R^0$ and $R^1$ have the same meanings as explained in connection with general formula I and R is a lower alkyl radical having 1 to 4 C-atoms, with hydrolytic splitting off of the residue R stereoselectively to give the correspondingly substituted 2(E), 4(E)-pentadienoic acid of the general formula VIII

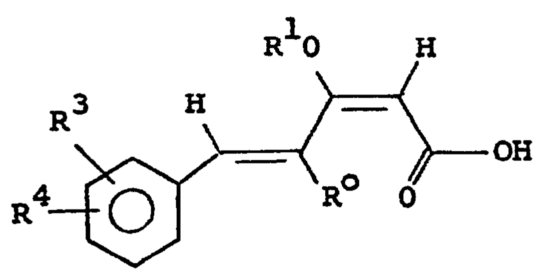

(VIII)

wherein $R^0$, $R^1$, $R^3$ and $R^4$ have the above-given meanings;

(B) the so resulting pentadienoic acid is converted into an ester of the general formula IX

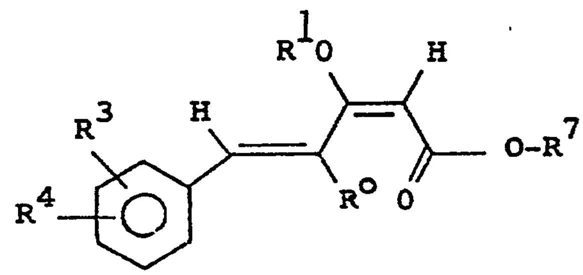

(IX)

wherein $R^7$ is a straight-chained or branched alkyl radical having 1 to 10 C-atoms or an aralkyl radical having 7 to 10 C-atoms,

(C) the ester of general formula IX is reacted by cis-Dihydroxylation by means of catalytic amounts of osmium tetroxide and of an oxidation agent to give the corresponding 3-alkoxy-4,5-(threo)-dihydroxy-5-($R^3$, $R^4$-subst.)-phenyl-2(E)-pentenoic acid ester of the general formula XI

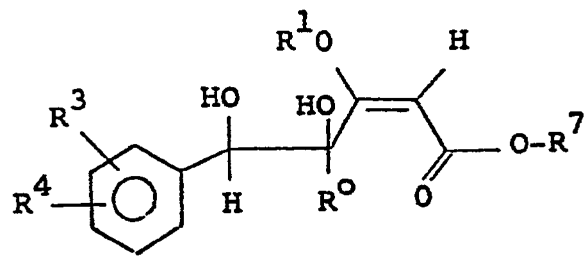

(XI)

(D) from which, with 1,4-elimination of the alcohol $R^7OH$, there is formed the corresponding threo-4-alkoxy-5[($R^3$,$R^4$-subst.)-phenylhydroxymethyl]-2(5H)-furanone of general formula I with $R^2 = H$;

(E) which subsequently is optionally reacted with a reactive compound of the general formula $R^2$–X, wherein $R^2$ has the same meaning as in general formula I with the exception of hydrogen, and X is a reactive group such as halogen or alkoxy, with the splitting off of X–H, to give the corresponding compound of general formula I with $R^2 \neq H$.

20. Process according to claim 19, wherein the pentadienoic acid of general formula VIII formed in step (A) is subjected to the cis-Dihydroxylation according to step (C) with the formation of the correspondingly substituted pentenoic acid of general formula XI with $R^7 = H$, whereafter the pentenoic acid is reacted according to step (D) with 1,4-elimination of $H_2O$ to give the correspondingly substituted furanone of general formula I, with $R^2 = H$, which is subsequently optionally reacted according to step (E) to give a compound of general formula I with $R^2 \neq H$.

21. Process according to claim 19 or 20, characterized in that the condensation of step (A) is carried out in a dipolar aprotic solvent and in basic medium.

22. Process according to claim 21, characterized in that the condensation is carried out in dimethyl sulphoxide with the addition of an alkali metal hydroxide, quaternary ammonium bases or mixtures thereof.

23. Process according to any one of claims 19 to 22, characterized in that the condensation is carried out under an inert gas atmosphere at a temperature of from about 70 to 140°C.

24. Process according to any one of claims 19 to 23, characterized in that in step (C), alkyl hydroperoxides, tertiary amine N-oxides or chlorates are used as an oxidation agent.

25. Process according to claim 24, characterized in that the dihydroxylation in step (C) is carried out in the presence of a quaternary ammonium base or of a quaternary ammonium salt.

26. Medicament, containing optionally together with a pharmacologically inert excipient, one or more of the compounds of general formula I, including those compounds of general formula I wherein $R^2$ is H or $CH_3$ when n = 0 or 2, $R^0 = H$, $R^1 = CH_3$, $R^3 = H$ and $R^4 = H$.

27. Use of the compounds of general formulae I, including those wherein $R^2$ is H or $CH_3$ when n = 0 or 2, $R^0 = H$, $R^1 = CH_3$, $R^3 = H$ and $R^4 = H$, and IX, including those wherein $R^0 = H$, $R^1$ = ethyl, $R^3 = H$, $R^4 = H$ and $R^7$ = ethyl, in the manufacture of anticonvulsives and anti-epileptics.

**Revendications**

1. 5-arylalkyl-4-alcoxy-2(5H)-furanones répondant à formule génerale I

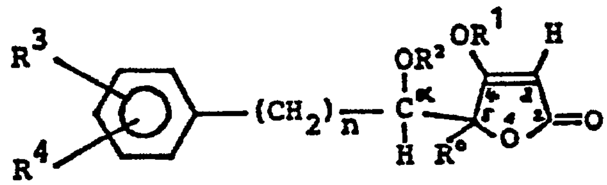

(I)

dans laquelle les atomes d'oxygène en C-5 et C-α prennent la position thréo l'un par rapport à l'autre, et dans laquelle
n désigne les nombres 0, 1 ou 2
$R^0$ designe un atome d'hydrogène ou un groupe alkyle inférieur en $C_1$–$C_3$,
$R^1$ désigne un groupe alkyle linéaire ou ramifié en $C_1$–$C_5$,
$R^2$ designe un atome d'hydrogène, un groupe alkyle inférieur en $C_1$–$C_3$ ou le radical

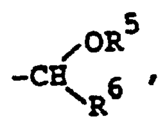

dans lequel
$R^5$ désigne un groupe alkyle inférieur en $C_1$–$C_5$, les groupes éthoxyéthyle ou méthoxyéthyle et
$R^6$ désigne un atome d'hydrogène, un groupe alkyle inférieur en $C_1$–$C_5$ ou le groupe méthoxyméthyle,
$R_3$ et $R_4$ désignent indépendamment l'un de l'autre un atome d'hydrogène, de fluor, de chlore ou de brome, un groupe alkyle inférieur en $C_1$–$C_3$, un groupe perfluoralkyle inférieur en $C_1$–$C_3$, les groupes difluorométhoxy ou nitro, à l'exclusion des composés répondant à la formule générale I, dans laquelle
$R^2$ désigne H ou $CH_3$, lorsque
n = 0 ou 2, $R^0$ = H, $R^1$ = $CH_3$, $R^3$ = H et $R^4$ = H.

2. 4-méthoxy-5-(phénylhydroxyméthyl)-2(5H)-furanones répondant a la formule générale II

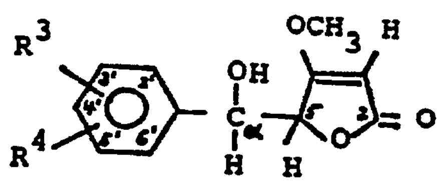

(II)

dans laquelle les atomes d'oxygène en C-5 et C-α prennent la posiiton thréo l'un par rapport à l'autre et dans laquelle l'un des deux radicaux $R^3$ et $R^4$ est un atome d'hydrogène et l'autre un atome de fluor, de chlore ou de brome se trouvant à la position 2′ ou désigne un groupe méthyle, trifluorométhyle ou nitro se trouvant à la position 2′.

3. 4-méthoxy-5-(phénylhydroxyméthyl)-2(5H)-furanones répondant à la formule générale II, dans laquelle les atomes d'oxygène en C-5 et C-α prennent l'un par rapport à l'autre la position thréo, et dans laquelle un des deux radicaux $R^3$ et $R^4$
– désigne un atome de fluor, de chlore ou de brome ou un groupe trifluorométhyle, à chaque fois en position 2′, et l'autre
– un atome de chlore ou de brome ou un groupe trifluorométhyle, à chaque fois en position 4′, 5′, ou 6′;

4. 4-méthoxy-5-[méthoxy-(phényl)-méthyl]-2(5H)-furanones répondant à la formule générale III

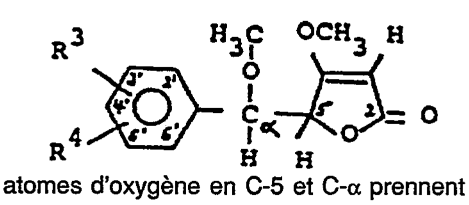

(III)

dans laquelle les atomes d'oxygène en C-5 et C-α prennent la position thréo l'un par rapport à l'autre et dans laquelle
$R^3$ et $R^4$ possèdent la signification indiquée dans la revendication 1, à l'exclusion des composés répondant à la formule générale III, dans laquelle les deux radicaux $R^3$ et $R^4$ désignent chacun un atome d'hydrogène:

5. 4-méthoxy-5-(phénylméthyl)-2(5H)-furanones répondant à la formule générale IV

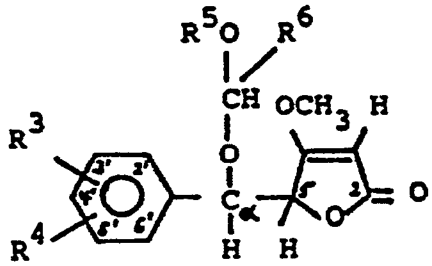

(IV)

dans laquelle les atomes d'oxygène en C-5 et C-α prennent l'un par rapport à l'autre la position thréo et dans laquelle
$R^5$ désigne un groupe méthyle ou méthoxyéthyle et
$R^3$, $R^4$ et $R^6$ possèdent la signification indiquée dans la revendication 1.

6. thréo-5-(2'-chlorophénylhydroxyméthyl)-4-méthoxy-2(5H)-furanone;
7. thréo-5-(2'-bromophénylhydroxyméthyl)-4-méthoxy-2(5H)-furanone;
8. thréo-5-(2'-fluororophénylhydroxyméthyl)-4-méthoxy-2(5H)-furanone;
9. thréo-5-(2'-trifluorométhyl-phényl-hydroxyméthyl)-4-méthoxy- 2(5H)-furanone;
10. thréo-5-(2',5'-dichlorophénylhydroxyméthyl)-4-méthoxy-2(5H)-furanone;
11. thréo-5-(2',4'-dichlorophénylhydroxyméthyl)-4-méthoxy-2(5H)-furanone;
12. Thréo-4-méthoxy-5-[méthoxyméthoxy-(2'-chlorophényl)-méthyl]-2(5H)-furanone;
13. Thréo-4-méthoxy-5-[méthoxyméthoxy-(2'-bromophényl)-méthyl]-2(5H)-furanone;
14. Thréo-4-méthoxy-5-[méthoxyméthoxy-(2'-fluorophényl)-méthyl]-2(5H)-furanone;
15. Thréo-4-méthoxy-5-[méthoxyméthoxy-(2'-trifluorométhyl-phényl)-méthyl]-2(5H)-furanone;
16. Thréo-4-méthoxy-5-[méthoxyméthoxy-(2'-chlorophényl)-méthyl]-2(5H)-furanone;
17. 3-alcoxy-5-(subst.)-phényl-2(E),4(E)-pentadiénoates répondant à la formule générale IX

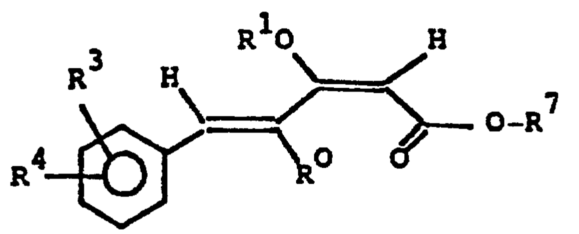

(IX)·

dans laquelle
$R^0$, $R^1$, $R^3$ et $R^4$ possèdent les significations indiquées dans la revendication 1 et
$R^7$ est un atome d'hydrogène, un groupe alkyle linéaire ou ramifié en $C_1$–$C_{10}$ ou un groupe aralkyle en $C_7$–$C_{10}$, à l'exception du composé dans lequel $R^0$ = H, $R^1$ = éthyle, $R^3$ = H, $R^4$ = H et $R^7$ = éthyle.

18. Composés selon la revendication 17, caractérisé en ce que $R^7$ est un atome d'hyrogène, un groupe alkyle linéaire ou ramifié en $C_1$–$C_4$ ou un groupe benzyle.

19. Procédé de préparation des composés selon la revendication 1 répondant à la formule générale I, dans laquelle n est 0, caractérisé par la combinaison des étapes opératoires suivantes:

(A) Un benzaldéhyde répondant à la formule générale VI

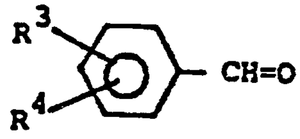

(VI)

dans laquelle $R^3$ et $R^4$ possèdent les significations indiquées à propos de l'explication de la formule I, est condensé avec un ester d'alkyle inférieur d'un acide 3-alcoxy-2(E)-alcénoïque répondant à la formule générale VII

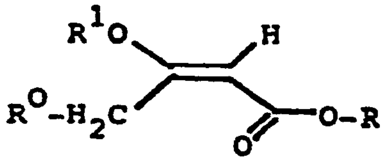

(VII)

dans laquelle $R^0$ et $R^1$ ont les significations indiquées à propos de l'explication de la formule générale I

et R est un groupe alkyle inférieur en $C_1$–$C_4$, avec coupure hydrolytique du radical R, d'une manière stéréosélective, pour donner l'acide 2(E),4(E)-pentadiénoïque substitué d'une manière correspondante, répondant à la formule générale VIII

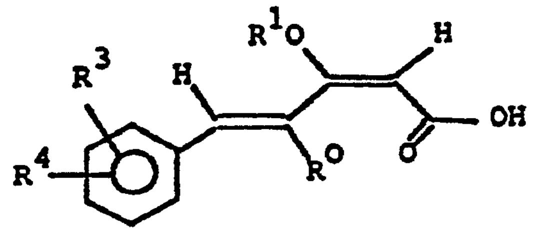

(VIII)

dans laquelle $R^0$, $R^1$, $R^3$ et $R^4$ possèdent les significations indiquées;

(B) l'acide pentadiénoïque ainsi formé est transformé en un ester répondant à la formule générale IX

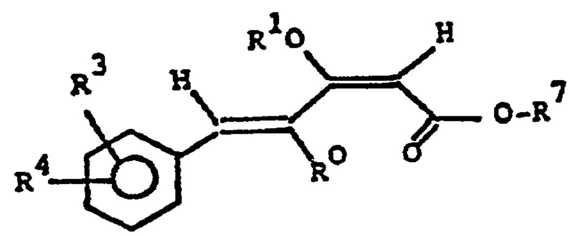

(IX)

dans laquelle $R^7$ est un groupe alkyle linéaire ou ramifié en $C_1$–$C_{10}$ ou un groupe aralkyle en $C_7$–$C_{10}$;

(C) l'ester répondant à la formule générale IX est transformé par cis-dihydroxylation au moyen de quantités catalytiques d'$OsO_4$ et d'un oxydant en l'ester de l'acide 3-alcoxy-4,5(thréo)-dihydroxy-5-(subst. par $R^3$, $R^4$)phényl-2(E)-penténoïque répondant à la formule générale XI

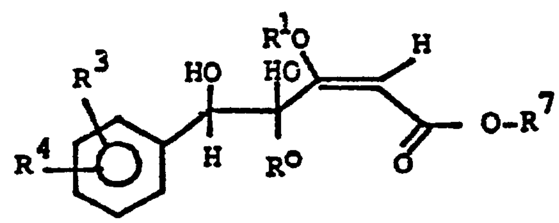

(XI)

(D) à partir duquel par 1,4-élimination de l'alcool $R^7OH$, il se forme la thréo-4-alcoxy-5-[(subst. par $R^3$,$R^4$) phényl-hydroxyméthyl]-2(5H)-furanone correspondante, répondant à la formule générale I dans laquelle $R^2$ = H,

(E) laquelle est ensuite mise à réagir avec un composé réactif répondant à la formule $R^2$–X, dans laquelle $R^2$ a la signification indiquée à propos de l'explication de la formule générale I, à l'exception de l'hydrogène, et

X est un groupe réactif, par exemple un atome d'halogène ou un groupe alcoxy, avec élimination de X–H, pour donner le composé correspondant répondant à la formule générale I dans laquelle $R^2$ est différent de H.

20. Procédé selon la revendication 19, caractérisé en ce que l'acide pentadiénoïque formé dans l'étape (A), répondant à la formule générale VIII, est soumis à la cis-dihydroxylation conformément à l'étape (C) avec formation de l'acide penténoïque substitué d'une manière correspondante, répondant à la formule générale XI dans laquelle $R^7$=H, après quoi l'acide penténoïque est mis à réagir conformément à l'étape (D) avec 1,4-élimination de $H_2O$, pour donner la furanone substituée d'une manière correspondante, répondant à la formule générale I avec $R^2$=H, laquelle est ensuite mise à réagir éventuellement, conformément à l'étape (E), pour donner un composé répondant à la formule générale I dans laquelle $R^2$ est différent de H.

21. Procédé selon la revendication 19 ou 20, caractérisé en ce que la condensation de l'étape (A) est effectué dans un solvant aprotique dipolaire et en milieu basique.

22. Procédé selon la revendication 21, caractérisé en ce que la condensation est effectuée dans du sulfoxyde de diméthyle avec addition d'un hydroxyde de métal alcalin, de bases ammonium quaternaire ou de melanges de ceux-ci.

23. Procédé selon l'une des revendications 19 à 22, caractérisé en ce que la condensation est effectuée sous atmosphère de gaz inerte, à une température comprise entre environ 70 et 140°C.

24. Procedé selon l'une des revendications 19 à 23, caractérisé en ce que dans l'étape (C), on utilise comme oxydant des hydroperoxydes d'alkyle, des N-oxydes d'amines tertiaires ou des chlorates.

25. Procédé selon la revendication 24, caractérisé en ce que la dihydroxylation dans l'étape (C) est effectuée en présence d'une base ammonium quaternaire ou d'un sel d'ammonium quaternaire.

26. Médicament contenant, éventuellement en même temps qu'un excipient pharmacologiquement inerte, un ou plusieurs des composés répondant à la formule générale I, y compris les composés répondant à la formule générale I dans laquelle $R^2$ désigne H ou $CH_3$, lorsque n = 0 ou 2 , $R^0$ = H, $R^1$ = $CH_3$ , $R^3$ = H et $R^4$ = H.

27. Utilisation des composés répondant aux formules générales I, y compris ceux dans lesquelles $R^2$ désigne H ou $CH_3$, lorsque n = 0 ou 2 , $R^0$ = H, $R^1$ = $CH_3$, $R^3$ = H et $R^4$ =H, et IX, y compris ceux dans lesquels $R^0$ = H, $R^1$ = éthyle, $R^3$ = H, $R^4$ = H et $R^7$ = éthyle pour la préparation de médicaments anti-convulsivants et anti-épileptiques.

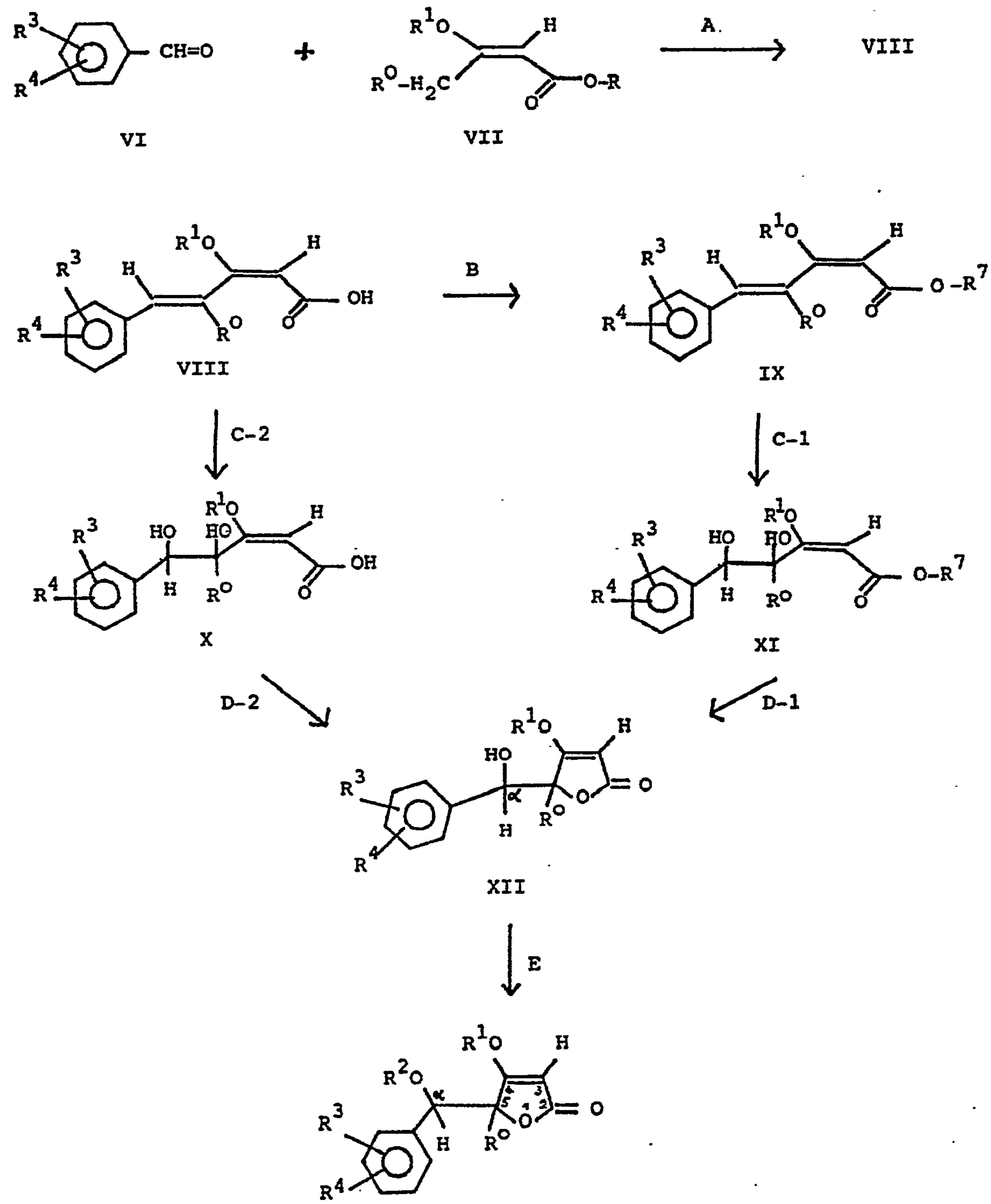


VI VII VIII

VIII IX

X XI

XII

XIII